# EUROPEAN PATENT SPECIFICATION

(11) **EP 2 841 109 B1**
(45) Date of publication and mention of the grant of the patent: **15.04.2026**
(21) Application number: 13719484.1
(22) Date of filing: 24.04.2013
(51) Int. Cl.: A61K 47/60, A61K 31/192

(54) **PRODRUGS OF HYDROXYL-COMPRISING DRUGS**
PRODRUGS VON HYDROXYL-HALTIGEN ARZNEIMITTELN
PRODROGUES DE MÉDICAMENTS COMPRENANT UN HYDROXYLE

(30) Priority: 25.04.2012 EP 12165516
(43) Date of publication of application: 04.03.2015
(73) Proprietor: Ascendis Pharma A/S, 2900 Hellerup (DK)
(72) Inventor: HERSEL, Ulrich, 69120 Heidelberg (DE); LEßMANN, Torben, 69120 Heidelberg (DE); RAU, Harald, 69120 Heidelberg (DE)
(74) Representative: Büchel, Edwin
(86) International application number: PCT/EP2013/058469
(87) International publication number: WO 2013/160340

(56) References cited:
- WO-A1-2011/089214
- WO-A1-2013/024052
- WO-A2-2009/095479
- US-A1- 2003 050 331
- SOHMA YOUHEI ET AL: "Development of water-soluble prodrugs of the HIV-1 protease inhibitor KNI-727: Importance of the conversion time for higher gastrointestinal absorption of prodrugs based on spontaneous chemical cleavage", JOURNAL OF MEDICINAL CHEMISTRY, AMERICAN CHEMICAL SOCIETY, US, vol. 46, no. 19, 11 September 2003 (2003-09-11), pages 4124 - 4135, XP002492718, ISSN: 0022-2623, [retrieved on 20030813], DOI: 10.1021/JM030009M

## Description

The present invention relates to a prodrug or a pharmaceutically acceptable salt thereof comprising a drug linker conjugate. The invention further relates to pharmaceutical compositions comprising said prodrugs and their use as medicaments.

To enhance physicochemical or pharmacokinetic properties of a drug in vivo such drug can be conjugated with a carrier. Typically, carriers in drug delivery are either used in a non-covalent fashion, with the drug physicochemically formulated into a solvent-carrier mixture, or by covalent attachment of a carrier reagent to one of the drug's functional groups.

However, the non-covalent approach requires a highly efficient drug encapsulation to prevent uncontrolled, burst-type release of the drug. Restraining the diffusion of an unbound, water-soluble drug molecule requires strong van der Waals contacts, frequently mediated through hydrophobic moieties and charged moieties for electrostatic binding. Many conformationally sensitive drugs, such as proteins or peptides, are rendered dysfunctional during the encapsulation process and/or during subsequent storage of the encapsulated drug. Furthermore, dependence of the release mechanism of the drug upon biodegradation may cause interpatient variability.

Alternatively, the drugs may be conjugated to a carrier via a transient linker molecule (carrier-linked prodrugs). This approach is applied to various classes of molecules, from so-called small molecules, through natural products up to larger proteins.

In order to ensure cleavage of the covalent bond between carrier and drug easy removal of said bond in vivo is required to release the drug (prodrug activation).

Prodrug activation may occur by enzymatic or non-enzymatic cleavage of the bond between the carrier and the drug molecule, or a sequential combination of both, e.g. an enzymatic step followed by a non-enzymatic rearrangement.

Enzymatically induced prodrug activation is characterized in that the cleavage in enzyme-free in-vitro environment such as an aqueous buffer solution, of, e.g., an ester or amide may occur, but the corresponding rate of hydrolysis may be much too slow and not therapeutically useful.

In an in-vivo environment, esterases or amidases are typically present and the esterases and amidases may cause significant catalytic acceleration of the kinetics of hydrolysis from twofold up to several orders of magnitude. Therefore, the cleavage is predominantly controlled by the enzymatic reaction.

A major drawback of predominantly enzymatic cleavage is interpatient variability. Enzyme levels may differ significantly between individuals resulting in biological variation of prodrug activation by the enzymatic cleavage. The enzyme levels may also vary depending on the site of administration. For instance, it is known that in the case of subcutaneous injection, certain areas of the body yield more predictable therapeutic effects than others. To reduce this unpredictable effect, non-enzymatic cleavage like for example by intramolecular catalysis is of particular interest.

Therefore, enzyme-independent autocatalytic cleavage of carrier and biologically active moiety is preferred. In most cases this is achieved by a suitably designed linker moiety between the carrier and the biologically active moiety, which is directly attached to the functional group of a biologically active moiety via a covalent bond.

Non-enzymatically autocatalytic cleavable prodrugs from amine-comprising drugs have been described for example in WO-A 2009/095479 and WO-A 2011/012722.

Y. Sohma et al., J. Med. Chem. 46 (2003), 4124-4135, describe ester based prodrugs, where the carrier is water-soluble and the biologically active moiety is derived from HIV-1 protease inhibitor KNI-727. The linker used is attached to the biologically active moiety via an ester group. The mechanism of this prodrug system is cyclization-activation by cyclic imide formation for the cleavage of ester bonds. However, this is disadvantageous because of the susceptibility of the ester functional group to enzymatic cleavage.

WO2011/089214A1 teaches prodrugs or pharmaceutically acceptable salt thereof, comprising a drug linker conjugate D-L, wherein D is a biologically active moiety or drug comprising an aromatic hydroxyl group that is conjugated to one or more polymeric carriers via carbamate containing linkers. Hydroxyl comprising drugs are a very specific subgroup of hydroxyl-comprising drugs. The linker moiety of WO2011/089214A1 was designed to function as a tool which by following an intramolecular cleavage of the carbamate bond achieves suitable release half-lives of aromatic hydroxyl drugs.

Thus, an object of the present invention is to provide such drug linker conjugates, where the reversible prodrug linker is covalently attached via a cleavable bond to a biologically active moiety (representing the drug after release), and where the linker is further covalently attached via a permanent bond to at least one carrier directly or via a spacer to form the carrier-linked prodrug.

This object is achieved with a prodrug as defined in the claims.

It was surprisingly found that such prodrugs at least partially overcome the abovementioned limitations.

The terms "drug" means any substance which can affect one or more physical or biochemical properties of a biological organism, including viruses, bacteria, fungi, plants, animals, and humans. In particular, as used herein, the term includes any substance intended for diagnosis, cure, mitigation, treatment, or prevention of disease in organisms, in particular humans or animals, or to otherwise enhance physical or mental well-being of organisms, in particular humans or animals. The term "biologically active moiety -D" refers to the part of a biologically active moiety-promoiety conjugate, which results after cleavage in a drug D-H of known biological activity. In general, the term "biologically active moiety" refers to a drug molecule which is connected to another moiety and in which drug molecule one or more atoms have been replaced with a linkage to said other moiety.

A "hydroxyl-comprising biologically active moiety" refers to a biologically active moiety which comprises a hydroxyl group, i.e. a moiety -OH**.** Said hydroxyl group may be connected to an aliphatic or aromatic moiety of the hydroxyl-comprising biologically active moiety.

"Free form" of a drug refers to the drug in its unmodified, pharmacologically active form, such as after being released from a prodrug.

"Prodrug" refers to any compound that undergoes biotransformation before exhibiting its pharmacological effects. Prodrugs can thus be viewed as biologically active moieties comprising specialized non-toxic protective groups used in a transient manner to alter or to eliminate undesirable properties in the parent molecule, i.e. the drug. This clearly also includes the enhancement of desirable properties in the drug and the suppression of undesirable properties.

The term "carrier-linked prodrug" refers to a prodrug that contains a reversible linkage of a given biologically active moiety with a transient carrier group that produces improved physicochemical or pharmacokinetic properties and that can be easily removed in vivo, usually by a hydrolytic cleavage.

The term "promoiety" refers to the part of the prodrug, which is not the biologically active moiety, thus meaning for example a polymeric carrier moiety and reversible prodrug linker moiety, respectively.

The term "reversible prodrug linker" refers to a moiety which on its one end is attached to a biologically active moiety through a reversible linkage and at another end is permanently attached to a polymeric carrier -Z. Such reversible prodrug linkers are non-enzymatically cleavable under physiological conditions (aqueous buffer at pH 7.4, 37°C) with half-lives ranging from one hour to three months. In the carrier-linked prodrugs of the present invention the reversible linkage between the prodrug linker L- and the biologically active moiety -D is an ester or carbamate.

Permanent linkages are non-enzymatically cleavable under physiological conditions (aqueous buffer at pH 7.4, 37°C) with half-lives of six months or longer, such as, for example, amides.

The term "polymer" describes a molecule comprising repeating structural units connected by chemical bonds in a linear, circular, branched, crosslinked or dendrimeric way or a combination thereof, which can be of synthetic or biological origin or a combination of both. It is understood that e.g. capping moieties may be present in a polymer. The term "polymeric" refers to a moiety comprising one or more polymer.

The term "hydrogel" refers to a three-dimensional, hydrophilic or amphiphilic polymeric network capable of taking up large quantities of water. Such network may be composed of homopolymers or copolymers and is insoluble due to the presence of covalent chemical or physical (ionic, hydrophobic interactions, entanglements) crosslinks. The crosslinks provide the network structure and physical integrity. Hydrogels exhibit a thermodynamic compatibility with water which allows them to swell in aqueous media. The chains of the network are connected in such a fashion that pores exist and that a substantial fraction of these pores are of dimensions between 1 nm and 1000 nm.

"C₁₋₆ alkyl" means an alkyl chain having 1 to 6 carbon atoms, e.g. if present at the end of a molecule: methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl, 3,3-dimethylpropyl; or e.g. -CH₂-, -CH₂-CH₂-, -CH(CH₃)-, -CH₂-CH₂-CH₂-, -CH(C₂H₅)-, -C(CH₃)₂-, when two moieties of a molecule are linked by the alkyl group. Each hydrogen of a C₁₋₆ alkyl carbon may be replaced by an optional substituent as further specified in the context of the optional substituents of -L¹-.

Accordingly, "C₁₋₂₀ alkyl" means an alkyl chain having 1 to 20 carbon atoms and "C₈₋₁₈ alkyl" means an alkyl chain having 8 to 18 carbon atoms. Accordingly, "C₁₋₅₀ alkyl" means an alkyl chain having 1 to 50 carbon atoms.

The term "C₁₋₂₀ heteroalkyl" in the context of the present invention denotes linear or branched alkyl chains wherein in said alkyl chains
- one or more carbon atom(s) is/are independently exchanged for a heteroatom or group of atoms selected from the group comprising -O-, -S-, -Si(R^{a})(R^{b})-, -C(O)O-, -C(O)-, -C(O)N(R^{a})-, -S(O)₂N(R^{a})-, -S(O)N(R^{a})-, -S(O)₂-, -S(O)-, N(R^{a})S(O)₂N(R^{b})-, -N(R^{a})-, -OC(O)-, -N(R^{a})C(O)-, -N(R^{a})S(O)₂-, -N(R^{a})S(O)-, -N(R^{a})C(O)O-, -N(R^{a})C(O)N(R^{b})-, -P(O)(OR^{a})O-, -OC(O)N(R^{a})-, -OP(O)(OR^{a})O-, -OP(O)(OR^{a})-, -CH=CH-, -C≡C-; and/or
- one or more hydrogen atom(s) is/are independently exchanged for -NR^{a}R^{b}, -CN, -Si(R^{a})(R^{b})R^{c}, -Cl, -F, -Br, -I, -OP(O)(OR^{a})OR^{b}, -P(O)(OR^{a})OR^{b}, -OP(O)(OR^{a})R^{b}, -OR^{a}, -OCO, -C(O)R^{a}, -C(O)N(R^{a})R^{b}, -OC(O)N(R^{a})R^{b}, -C(O)OR^{a}, -OC(O)R^{a}, -SR^{a}, -S(O)₂N(R^{a})R^{b}, -S(O)N(R^{a})R^{b}, -S(O)₂R^{a}, -S(O)R^{a}, -N(R^{a})S(O)R^{b}, -N(R^{a})S(O)₂R^{b}, -N(R^{a})S(O)₂N(R^{b})R^{c}, -N(R^{a})C(O)R^{b}, -N(R^{a})C(O)OR^{b}, -N(R^{a})C(O)N(R^{b})R^{c}, -CH=CH₂, -C≡CH;
   wherein -R^{a}, -R^{b} and -R^{c} are independently selected from the group consisting of -H, C₁₋₆ alkyl, C₂₋₆ alkenyl and C₂₋₆ alkynyl;

and wherein said alkyl chains comprise 1 to 20 carbon atom(s) and/or heteroatom(s) selected from the group of heteroatoms consisting of O, S, N, P, and Si, provided that at least one carbon is present in said alkyl chain. Each hydrogen of a C₂₋₂₀ heteroalkyl may be replaced by an optional substituent as further specified in the context of the optional substituents of -L¹-. The terms "C₁₋₁₀ heteroalkyl" and "C₁₋₅₀ heteroalkyl" are defined accordingly and comprise alkyl chains of 1 to 10 and 2 to 50 carbon atom(s) and/or heteroatom(s) selected from the group of heteroatoms consisting of O, S, N, P and Si, respectively.
"C₂₋₆ alkenyl" means a branched or unbranched alkenyl chain having 2 to 6 carbon atoms, e.g. if present at the end of a molecule: -CH=CH₂, -CH=CH-CH₃-, -CH₂-CH=CH₂-, -CH=CHCH₂-CH₃-, -CH=CH-CH=CH₂ or e.g. -CH=CH-, when two moieties of a molecule are linked by the alkenyl group. Each hydrogen of a C₂₋₆ alkenyl carbon may be replaced by an optional substituent as further specified as further specified in the context of the optional substituents of -L¹-. Accordingly, the term "alkenyl" relates to a carbon chain with at least one carbon carbon double bond. Optionally, one or more triple bonds may occur.

"C₂₋₆ alkynyl" means a branched or unbranched alkynyl chain having 2 to 6 carbon atoms, e.g. if present at the end of a molecule: -C=CH, -CH₂-C=CH, -CH₂-CH₂-C=CH, -CH₂-C=C-CH₃, or e.g. -C=C- when two moieties of a molecule are linked by the alkynyl group. Each hydrogen of a C₂₋₆ alkynyl carbon may be replaced by an optional substituent as further specified in the context of the optional substituents of -L¹-. Accordingly, the term "alkynyl" relates to a carbon chain with at least one carbon carbon triple bond. Optionally, one or more double bonds may occur.

"C₃₋₇ cycloalkyl" means a cyclic alkyl chain having 3 to 7 carbon atoms, which may have carbon-carbon double bonds being at least partially saturated, e.g. cyclopropyl, cyclobutyl, cyclopentyl, cyclohexyl, cyclohexenyl, cycloheptyl. Each hydrogen of a cycloalkyl carbon may be replaced by a substituent as further specified in the context of the optional substituents of -L¹-. The term "C₃₋₇ cycloalkyl" also includes bridged bicycles like norbonane or norbonene.

Accordingly, "C₃₋₁₀ cycloalkyl" means a cyclic alkyl having 3 to 10 carbon atoms, e.g. C₃₋₇ cycloalkyl, cyclopropyl, cyclobutyl, cyclopentyl, cyclohexyl, cyclohexenyl, cycloheptyl, cyclooctyl, cyclononyl, cyclodecyl. The term "C₃₋₁₀ cycloalkyl" also includes at least partially saturated carbomono- and -bicycles. Each hydrogen of a C₃₋₇ cycloalkyl carbon may be replaced by a substituent as further specified in the context of the optional substituents of -L¹-.

"4- to 7-membered heterocyclyl" or "4- to 7-membered heterocycle" means a ring with 4, 5, 6 or 7 ring atoms that may contain up to the maximum number of double bonds (aromatic or non-aromatic ring which is fully, partially or unsaturated) wherein at least one ring atom up to 4 ring atoms are replaced by a heteroatom selected from the group consisting of sulfur (including - S(O)-, -S(O)₂-), oxygen and nitrogen (including =N(O)-) and wherein the ring is linked to the rest of the molecule via a carbon or nitrogen atom. Examples for a 4- to 7-membered heterocycles are azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine or homopiperazine. Each hydrogen of a 4- to 7-membered heterocyclyl carbon may be replaced by a substituent as further specified in the context of the optional substituents of -L¹-.

"9- to 11-membered heterobicyclyl" or "9- to 11-membered heterobicycle" means a heterocyclic system of two rings with 9 to 11 ring atoms, where at least one ring atom is shared by both rings and that may contain up to the maximum number of double bonds (aromatic or non-aromatic ring which is fully, partially or un-saturated) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a 9 to 11 membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine or pteridine. The term 9- to 11-membered heterobicycle also includes spiro structures of two rings like 1,4-dioxa-8-azaspiro[4.5]decane or bridged heterocycles like 8-aza-bicyclo[3.2.1]octane. Each hydrogen of a 9- to 11-membered heterobicyclyl carbon may be replaced by a substituent as further specified in the context of the optional substituents of -L¹-.

"Halogen" means fluoro, chloro, bromo or iodo. It is generally preferred that halogen is fluoro or chloro.

Various moieties are written in a simplified manner instead of drawing their full structure. The term "-N(R^{a})C(O)N(R^{b})-" for example means a moiety of the following structure: 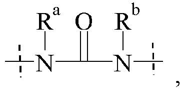 meaning that atoms or groups of atoms written in brackets are not part of the main chain, but extend from the first atom to their left without brackets. Similarly, the term "-N(R^{a})S(O)₂R^{b}" refers to a moiety of the following structure:

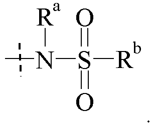

The term "interrupted" means that between two carbon atoms an atom or group as further specified is inserted. Such atom or group inserted between two carbon atoms can be in either orientation, even if only one orientation is listed.

The term "substituted" means that one or more -H atoms of a molecule are replaced by a different atom or a group of atoms.

In general, the terms "comprise" or "comprising" also encompasses "consist of" or "consisting of'.

Preferably, -Y- of formula (I) is -N(R¹)-.

Preferably, -X²- of formula (I) is -C(R⁷)(R^{7a})-.

Preferably, -R¹ of formula (I) is methyl.

Preferably, -R⁴ and -R^{4a} of formula (I) are both methyl.

Preferably, -Y- of formula (I) is -N(R¹)- and -X- of formula (I) is -C(R⁴)(R^{4a})-.

More preferably, -L¹- has the structure of formula (II): 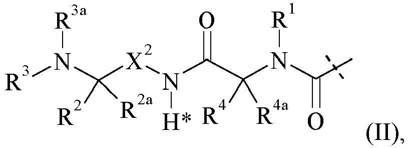
wherein the dashed line indicates attachment to a hydroxyl group of -D by forming a carbamate bond, and
-R¹, -R², -R^{2a}, -R³, -R^{3a}, -R⁴ and -R^{4a} are independently selected from the group consisting of -H and C₁₋₆ alkyl.

Preferably, -R¹, -R⁴ and -R^{4a} of formula (I) or (II) are independently selected from the group consisting of -H and methyl.

Most preferably, -L¹- has the structure of one of formulas (i) to (xxxxxiv): 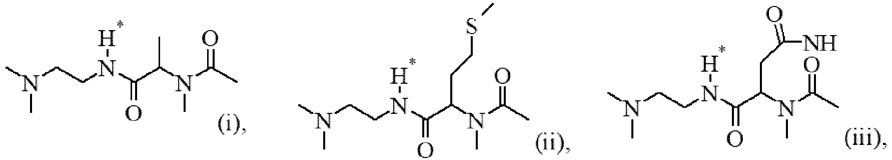 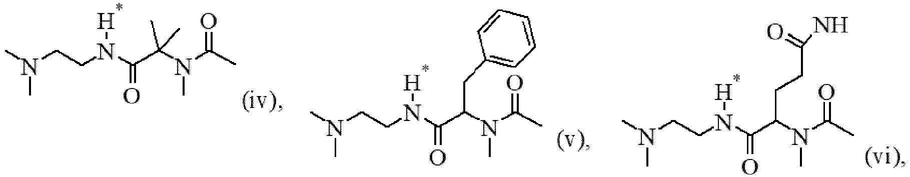 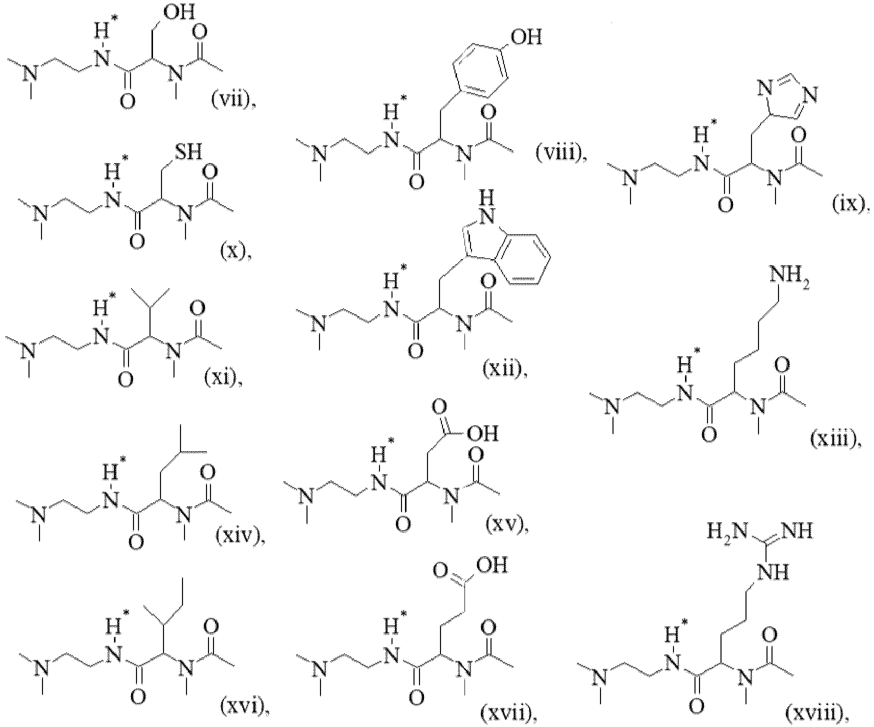 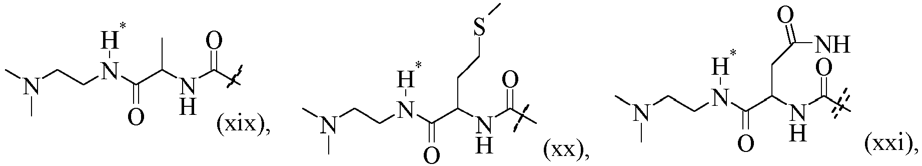 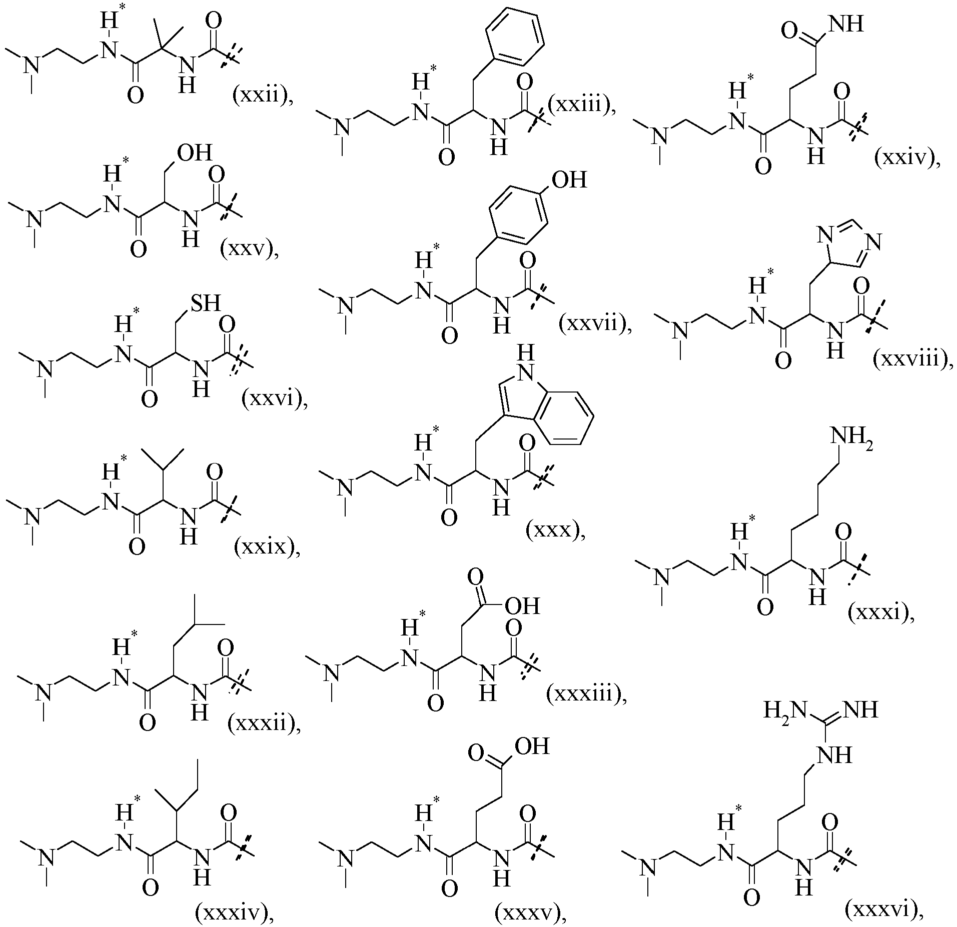 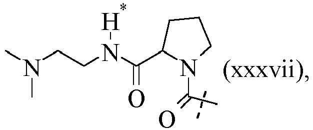 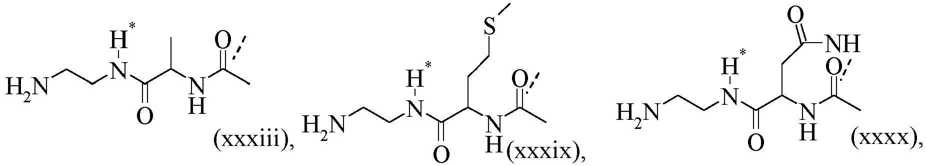 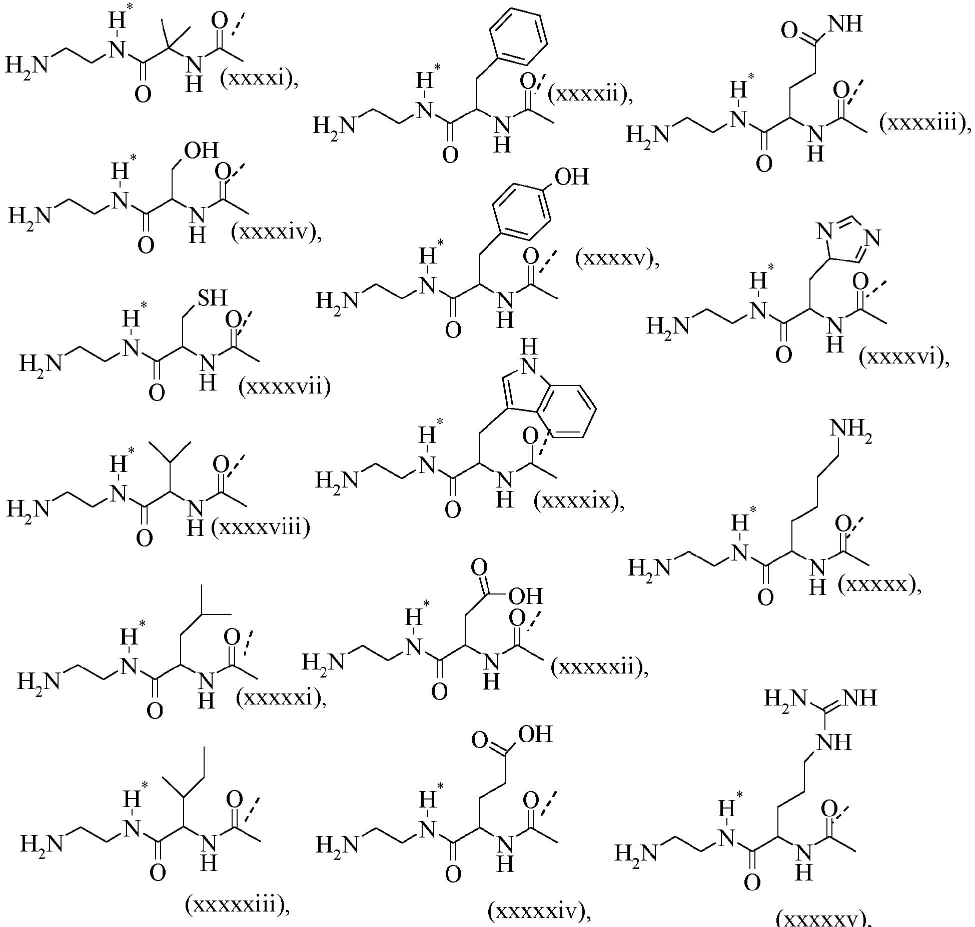 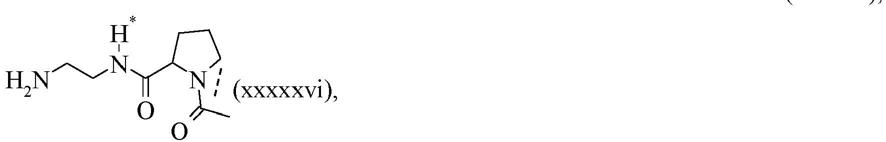 wherein the dashed line indicates attachment to a hydroxyl group of -D by forming a carbamate bond.

One hydrogen of formula (I), (II), (i) to (xxxxxiv) is replaced by a group -L²-Z.

In general, -L²- can be attached to -L¹- at any position apart from the replacement of the hydrogen marked with an- asterisk in formula (I), (II), and (i) to (xxxxxvi). Preferably, one hydrogen given by -R¹ to -R^{8a} directly or as hydrogen of the C₁₋₆ alkyl and rings given by the definition of -R¹ to -R^{8a} is replaced by -L²-Z.

Preferably, a moiety -L²- is attached to -L¹- via -Y-, -X-, -X²-, -R², -R^{2a}, -R³ or -R^{3a}.

Preferably, -L¹- is substituted at -R³ with one moiety -L²-Z.

Furthermore, -L¹- may be optionally further substituted. In general, any substituent may be used as far as the cleavage principle is not affected. Preferably, one or more further optional substituents of -L¹- are independently selected from the group consisting of C₁₋₆ alkyl, C₂₋₆ alkenyl, C₂₋₆ alkynyl, C₂₋₂₀ heteroalkyl, halogen and -OH.

-L²- is a single chemical bond or a spacer. In case -L²- is a spacer, it is preferably C₁₋₅₀ heteroalkyl, which is optionally interrupted with one or more T and which is substituted with -Z;
wherein
T is phenyl, naphthyl, indenyl, indanyl, tetralinyl, C₃₋₁₀ cycloalkyl, 4- to 7-membered heterocyclyl or 9- to 11-membered heterobicyclyl, wherein T is optionally substituted with one or more -R⁹, which are the same or different; and
-R⁹ is halogen, -CN, oxo (=O), -C(O)OH, -OH, -S(O)₂NH₂, -S(O)NH₂, -S(O)₂OH, -S(O )OH, -SH, -NH₂, -NO₂, C₁₋₆ alkyl or C₁₋₁₀ heteroalkyl.

More preferably, -L²- is C₁₋₂₀ alkyl or C₁₋₂₀ heteroalkyl.

Preferably, -L²- has a molecular weight ranging from 14 g/mol to 750 g/mol.

Preferably, -L²- is attached to -Z via the terminal group

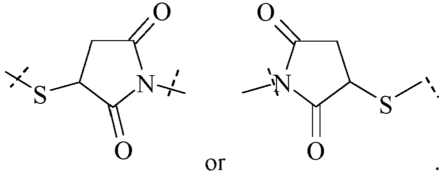

In case -L²- has such terminal group it is furthermore preferred that -L²- has a molecular weight ranging from 14 g/mol to 500 g/mol calculated without such terminal group.

In one embodiment the polymeric carrier -Z is a C₈₋₁₈ alkyl group.

Preferably, the polymeric carrier -Z is a polymer having a molecular weight of 0.5 to 160 kDa, more preferably of 1 to 120 kDa, even more preferably of 5 to 100 kDa, even more preferably of 10 to 80 kDa, even more preferably of 10 to 70 kDa and most preferably of 20 to 60 kDa.

Preferably, the polymeric carrier -Z comprises at least one of the polymers selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans and copolymers thereof.

Preferably, -Z is a protein.

Preferably, -Z is a protein selected from the group consisting of albumin, transferrin, and immunoglobulin.

Preferably, -Z is a protein carrier as disclosed in EP11177408.

Preferably, -Z is a linear or branched poly(ethylene glycol) with a molecular weight from 2,000 Da to 150,000 Da.

Preferably, -Z is a PEG carrier as disclosed in EP11177405 and EP11177406.

Preferably, -Z is a hydrogel, more preferably a PEG-based hydrogel and most preferably a hydrogel as disclosed in WO-A 2006/003014 or WO-A 2011/012715.

Preferably, D-H is a small molecule bioactive agent or a biopolymer.

Preferably, D-H is a biopolymer selected from the group of biopolymers consisting of proteins, polypeptides, oligonucleotides, and peptide nucleic acids.

Preferably, D-H is a protein prepared by recombinant DNA technologies.

Preferably, D-H is a small molecule drug, more preferably D-H is a small molecule drug selected from the group comprising: (-)-(2R*,3R*,11bS*)-Dihydrotetrabenazine, (-)-(2R*,3S*,11bR*)-Dihydrotetrabenazine, (-)-2-(2-Bromohexadecanoyl)paclitaxel, (-)-9-Dehydrogalanthaminium bromide, (-)-Calanolide B, (-)-Calicheamicinone, (-)-Cicloprolol, (-)-cis-Resorcylide, (-)-Conophylline, (-)-Desoxyepothilone B, (-)-Dihydrocalanolide B, (-)-Epigallocatechin gallate, (-)-Epothilone A, (-)-Epothilone E, (-)-Gossypol, (-)-Indocarbazostatin B, (-)-Kendomycin, (-)-Kolavenol ((-)-enantiomer), (-)-Nebivolol, (-)-Pironetin, (-)-Salbutamol hydrochloride, (-)-Salmeterol, (-)-Ternatin, (-)-Vexibinol, (+)-(2R*,3R*,11bS*)-Dihydrotetrabenazine, (+)-(2R*,3S*,11bR*)-Dihydrotetrabenazine, (+)-(S)-Hydroxychloroquine, (+)-23,24-Dihydrodiscodermolide, (+)-Almuheptolide A, (+)-alpha-Viniferin, (+)-Amphidinolide T1, (+)-Azacalanolide A, (+)-Calanolide A, (+)-Calanolide B, (+)-Cystothiazole B, (+)-Decursinol, (+)-Dihydrocalanolide A, (+)-Discodermolide, (+)-Erythrodiol, (+)-Etorphine, (+)-Genipin, (+)-Hemipalmitoylcarnitinium, (+)-Indocarbazostatin, (+)-Isamoltan, (+)-Lasonolide A, (+)-Scyphostatin, (+)-Sotalol, (E)-9,10-Didehydroepothilone D, (R)-Albuterol hydrochloride, (R)-Almokalant, (R)-Bicalutamide, (R)-Gossypol, (R)-Sulfinosine, (S)-(+)-Curcuphenol, (S)-Almokalant, (S)-Methylnaltrexone bromide, (S)-Oxiracetam, (S)-Sotalol, (S)-Sulfinosine, [125I]-Iodomethyllycaconitine, [8]-Gingerol, [99Tc]Demobesin 3, [99Tc]Demobesin 4, [Ala11,D-Leu15]Orexin B, [Arg(Me)9] MS-10, [D-Tyr1,Arg(Me)9] MS-10, [D-Tyr1] MS-10, [N-MeIle4]-cyclosporin, [Trp19] MS-10, 1,24(R)-Dihydroxyvitamin D3, 11-Hydroxyepothilone D, 12'-Methylthiovinblastine dihydrochloride, 13-Deoxyadriamycin hydrochloride, 13-Deoxydoxorubicin hydrochloride, 14beta-Hydroxydocetaxel, 14beta-Hydroxydocetaxel-1,14-acetonide, 14beta-Hydroxytaxotere, 14-C-Methyltriptolide, 14-Hydroxyclarithromycin, 14-Methoxymetopon, 14-Phenylpropoxymetopon, 14-Pivaloylandrographolide, 15-Methylepothilone B, 16-Aza-epothilone B, 16-Methyloxazolomycin, 17beta-Estradiol, 17beta-Hydroxywortmannin, 17-Deacetylnorgestimate, 17-Methyltestosterone, 18,19-Dehydrobuprenorphine hydrochloride, 18-Hydroxycoronaridine, 19-O-Demethylscytophycin C, 19-O-Methylgeldanamycin, 1alpha,25-Dihydroxyvitamin D3-23,26-lactone, 1-alpha-Hydroxyvitamin D3, 1alpha-Hydroxyvitamin D4, 1-Deoxynojirimycin, 2,12-Dimethyleurotinone, 21-Aminoepothilone B, 21-Hydroxyepothilone D, 22-Ene-25-oxavitamin D, 22-Oxacalcitriol, 24(S)-Ocotillol, 24-Deoxyascomycin, 25-Anhydrocimigenol-3-O-beta-D-xylopyranoside, 26-Fluoroepothilone, 2-Aminoaristeromycin, 2-Aminoneplanocin A, 2'-Hydroxymatteucinol, 2-Methoxyestradiol, 2'-Palmitoylpaclitaxel, 3,7a-Diepialexine, 36-Dihydroisorolliniastatin 1, 3-Allyl farnesol, 3-Angeloylingenol, 3beta-Erythrodiol, 3-Bromodiosmetine, 3-Bromodiosmine, 3-Chlorodiosmetine, 3-Chlorodiosmine, 3-Deazaadenosine, 3-Epimaxacalcitol, 3-Ingenyl angelate, 3-O-Methylquercetin, 4,6-diene-Cer, 4',7,8-Trihydroxyisoflavone, 41-Demethylhomooligomycin B, 44-Homooligomycin B, 4-Chlorophenylthio-DADMe-immucillin-A, 4-Demethylepothilone B, 4-Demethylpenclomedine, 4'-epi-Adriamycin, 4'-epiDoxorubicin, 4'-Ethynylstavudine, 4'-Galactosyllactose, 4-Hydroxyandrostenedione, 4"-Hydroxymevastatin lactone, 5(R)-Hydroxytriptolide, 5,4'-Diepiarbekacin, 5,6-Dehydroascomycin, 5-Aza-2'-deoxycytidine, 5-azacitidine, 5'-Epiequisetin, 5-Ethylthioribose, 5-Fluorodeoxyuridine, 5'-Homoneplanocin A, 5-Iodofredericamycin A, 5-Phenylthioacyclouridine, 5Z-7-Oxozeaenol, 6alpha-7-Epipaclitaxel, 6'-Homoneplanocin A, 6-Hydroxyscytophycin B, 6-O-Methylerythromycin A, 7,7a-Diepialexine, 7-Deoxytaxol, 7-Methylcapillarisin, 7-Monohydroxyethylrutoside, 8(R)-Fluoroidarubicin hydrochloride, 8',9'-Dehydroascochlorin, 8-Prenylapigenin, 8-Prenylnaringenin, 9,11-Dehydrocortexolone 17alpha-butyrate, 9,9-Dihydrotaxol, 9-Aminocamptothecin, 9-Hydroxycrisamicin A, 9-Hydroxyrisperidone, 9-Nitrocamptothecin, Abacavir sulfate, Abaperidone hydrochloride, Abarelix, Abietaquinone methide, Abiraterone, Acacetin, Acadesine, Acarbose, Acaterin, Acebutolol hydrochloride, Aceneuramic acid sodium salt, Acetamidoxolutamide, Acetaminophen, Achimillicic acid A lactone, Aciclovir, Aclacinomycin A, Aclacinomycin-X, Aclarubicin, Acolbifene hydrochloride, Acomustine, Acotiamide hydrochloride hydrate, Acrovestone, Actinoplanone A, Actinoplanone B, Aculeacin Agamma, Acycloguanosine, Acyclovir, Acyline, Adamantyl globotriaosylceramide, Adaphostin, Adecypenol, Adelmidrol, Adenallene, Adenophostin A, Adenophostin B, Adenosine, Adlupulon, Aerothricin 1, Aerothricin 16, Aerothricin 41, Aerothricin 45, Aerothricin 50, Aerothricin 55, Afeletecan hydrochloride, Agelasphin 517, Agelasphin 564, Aglaiastatin A, Aglaiastatin C, Aglepristone, Aica-riboside, Albaconazole, Albifylline, Albocycline K3, Albuterol nitrate, Albuterol sulfate, Alchemix, Alclometasone dipropionate, Alcuronium chloride, Aldecalmycin, Aldifen, Alemcinal, Alendronate sodium, Alendronic acid sodium salt, Alfacalcidol, Alfatrinositol, Alisamycin, Alkasar-18, Allixin, All-trans-Fucoxanthin, Almokalant, Almuheptolide A-(+), alpha-Calcidol, alpha-C-Galactosylceramide, alpha-Flupenthixol hydrochloride (cis-isomer), alpha-Galactosylceramide, alpha-Galactosylceramide-BODIPY, alpha-Lactosylceramide, alpha-Mangostin, alpha-Methyl epinephrine, alpha-Methylnorepinephrine, alpha-Peltatin ((-)-enantiomer), alpha-Pyrone I, alpha-Sialosylcholesterol sodium salt, Alprafenone hydrochloride, Alprenolol hydrochloride, Altohyrtin A, Altorhyrtin C, Altromycin A, Altromycin B, Altromycin C, Altromycin D, Altromycins, Alvespimycin hydrochloride, Alvocidib hydrochloride, Amamistatin A, Amamistatin B, Amarogentin, Amelometasone, Amelubant, Amibegron hydrochloride, Amidox, Amikacin, Aminocandin, Aminosidine, Ammocidin, Ammocidin A, Amosulalol hydrochloride, Amphidinolide E, Amphidinolide T1, Amphinidin A, Amprenavir, Amrubicin hydrochloride, Amurensin H, Amycomycin, Anandamide, Androstanolone, Androxolutamide, Anecortave acetate, Anguillosporal, Anguinomycin C, Anguinomycin D, Anidulafungin, Ankinomycin, Annamycin, Annocherimolin, Annomolin, Annulin C, Ansamycin, Antide, Antide-2, Antide-3, Antimycin A11, Antimycin A12, Antimycin A13, Antimycin A14, Antimycin A15, Antimycin A16, Antocin II, Apadenoson, Apalcillin sodium, Apaziquone, Aphidicolin, Aphidicolin glycinate, Apicularen A, Apicularen B, Apigenin, Aplidine, Apomine, Apomorphine hydrochloride, Apricitabine, Aragusterol A, Aragusterol C, Aranorosin, Aranorosinol A, Aranorosinol B, Aranose, Aranoza, Arbekacin, Arbekacin sulfate, Arbidol, Arborcandin A, Arborcandin B, Arborcandin C, Arborcandin D, Arborcandin E, Arborcandin F, Arbutamine hydrochloride, Archazolid A, Archazolid B, Archazolide A, Argiopine, Argiotoxin-636, Arisostatin A, Arotinolol hydrochloride, Arteminolide B, Arteminolide C, Arteminolide D, Arzoxifene hydrochloride, Asiaticosid, Asiaticoside, Asimadoline, Asperlicin B, Asperlicin E, Astringinine, Astromicin sulfate, Atalaphillidine, Atalaphillinine, Atazanavir sulfate, Atenolol, Atigliflozin, Atorvastatin calcium, Atosiban, Atovaquone, Atraric acid, Atrinositol, Aureobasidin A, Auristatin E, Aurothioglucose, Australifungin, Australine, Avicenol A, Avicequinone A, Avicin D, Avicin G, Avorelin, Axitirome, Azacitidine, Azacytidine, Azaresveratrol, Azatoxin, Azidothymidine, Azithromycin, Azithromycin copper complex, Azodisal disodium, Bactobolin, Bafilomycin A1, Baicalein, Balsalazide disodium, Bambuterol, Banoxantrone, Baogongteng A, Barixibat, Basifungin, Bazedoxifene hydrochloride, Becatecarin, Beciparcil, Beclometasone dipropionate, Beclomethasone dipropionate, Becocalcidiol, Bedoradrine sulfate, Befloxatone, Befunolol hydrochloride, Begacestat, Belactin B, Belotecan hydrochloride, Beloxepin, Benadrostin, Benastatin C, Benastatin D, Benexate cyclodextrin, Benflumetol, Bengazole A, Bengazole B, Benzbromaron, Benzbromarone, Benzhexol hydrochloride, Benzimidavir, Beraprost sodium, Berefrine, Berlafenone hydrochloride, Bervastatin, beta-Ecdysterone, beta-Hydroxy beta-methylbutyrate, beta-Mangostin, Betamethasone butyrate propionate, Betamethasone dipropionate, beta-Sialosylcholesterol sodium salt, beta-Tigogenin cellobioside, Betaxolol hydrochloride, Bevantol hydrochloride, Bevantolol hydrochloride, Biapenem, Bicalutamide, Biemnidin, Bimatoprost, Bimoclomol, Bimoclomol 1-oxide, Binodenoson, Biochanin A, Bioxalomycin alpha 1, Bioxalomycin alpha2, Biperiden, Bipranol hydrochloride, Bisabosqual A, Bisabosqual B, Bisabosqual C, Bisabosqual D, Bisphenol, Bitolterol mesylate, Bizelesin, Bleomycin A2 sulfate, Boforsin, Bohemine, Boholmycin, Bolinaquinone, Bosentan, Brandisianin B, Brandisianin C, Brasilinolide B, Brecanavir, Breflate, Breynin A, Breynin B, Brivudine, Bromocriptine mesilate, Bromohydrin pyrophosphate, Bromotopsentin, Bromovinyldeoxyuridine, Brovavir, Bryostatin 1, Bryostatin 10, Bryostatin 11, Bryostatin 12, Bryostatin 13, Bryostatin 9, Budesonide, Buflomedil pyridoxalphosphate, Buprenorphine hydrochloride, Burefrine, Butalactin, Butein, Buteranol, Butixocort, Butixocort propionate, Butofilolol, Byssochlamysol, Cabazitaxel, Cadralazine, Calanolide A, Calanolide B, Calcipotriene, Calcipotriol, Calcitriol, Calebin A, Calicheamicin gamma1 aglycone, Calocoumarin A, Calphostin A, Calphostin B, Calphostin C, Calphostin D, Calphostin I, Calteridol calcium, Cambrescidin 800, Cambrescidin 816, Cambrescidin 830, Cambrescidin 844, Camiglibose, Campestanol ascorbyl phosphate, Candelalide B, Candelalide C, Canrenoate potassium, Canventol, Capadenoson, Capecitabine, Capillarisin, Capridine beta, Capsazepine, Carabersat, Carbazomadurin A, Carbazomadurin B, Carbazomycin G, Carbazomycin H, Carbetocin, Carbovir, Caribaeolin, Caribaeoside, Carisbamate, Carmoterol hydrochloride, Carpesterol, Carquinostatin A, Carsatrin, Carteolol hydrochloride, Carteramine A, Carvastatin, Carvedilol, Cassigalol A, Castanospermine, Catechaldehyde, Cefbuperazone sodium, Cefonicid sodium, Cefoperazone sodium, Cefpiramide sodium, Celgosivir, Celikalim, Cephalostatin 1, Cephalostatin 2, Cephalostatin 3, Cephalostatin 4, Cephalostatin 7, Cephalostatin 8, Cephalostatin 9, Cerebroside A, Cerebroside B, Cerebroside C, Cerebroside D, Cerivastatin sodium, Cethromycin, Chaetoatrosin A, Chaetocin, Chafuroside, Chetocin, Chloptosin, Chlorazicomycin, Chlorofusin, Chlorogentisylquinone, Chlortalidone, Chlortenoxicam, Chlorthalidone, Choline alfoscerate, Choline fenofibrate, Choline glycerophosphate, Choline thioctate, Chondramide A, Chondramide B, Chondramide C, Chondramide D, Ciclesonide, Cicletanine, Ciclopiroxolamine, Ciclosporin, Cidofovir, Cimaterol, Cimetidine bismuth citrate, Cimetidine bismuth L-tartrate, Cimetropium bromide, Cinnabaramide A, Cinolazepam, Ciprokiren, cis-6-Shogaol, Citpressine I, Citreamicin-alpha, Citreamicin-eta, Citropeptin, Citrusinine-I, Cladribine, Clarithromycin, Clausenamine A, Clavarinone, Clavulanate potassium, Clavulanic acid potassium salt, Clazosentan, Clevudine, Clidinium bromide, Clindamycin hydrochloride, Clinprost, Clioquinol, Clitocine, Clobenoside, Clofarabine, Cloranolol hydrochloride, Clostomycin B1, Cocositol, Colabomycin A, Coleneuramide, Coleonol, Coleophomone B, Colestilan, Colestilan chloride, Colestimide, Colforsin, Colforsin daproate hydrochloride, Colforsin dapropate hydrochloride, Colforsin daropate hydrochloride, Colupulon, Combretastatin A-1, Combretastatin A-2, Combretastatin A-3, Combretastatin B-1, Combretastatin B-2, Combretastatin B-3, Combretastatin B-4, Combretastatin D-1, Combretastatin D-2, coniferol alcohol, Coniosetin, Conocurvone, Conophylline, Contignasterol, Contortumine hydrochloride, Coproverdine, Correolide, Cortexolone 17alpha-propionate, Corylifolinin, Corynecandin, Cositecan, Costatolide, Coumamidine gamma1, Coumamidine gamma2, Covidarabine, Crassicauline A, Crellastatin A, Crisamicin C, Crisnatol mesilate, Crobenetine, Cromakalim, Cromoglycate sodium, Cromolyn sodium, Crossoptine A, Crossoptine B, Cucoline, Curtisian A, Curtisian B, Curtisian D, Curvularol, Cyanidin, Cyanidin chloride monohydrate, Cyanidol chloride, Cyclamenol, Cyclandelate, Cyclipostin A, Cyclocommunol, Cyclohexanediol, Cyclomarin A, Cyclooctatin, Cycloplatam, Cyclosporin, Cyclosporin A, Cyclosporin J, Cyclosporine, Cyclosporine A, Cyclotheonamide A, Cygalovir, Cyrtominetin, Cystocin, Cystothiazole C, Cystothiazole D, Cystothiazole F, Cytallene, Cytarabine, Cytarabine ocfosfate, Cytaramycin, Cytoblastin, Cytochalasin B, Cytochlor, Cytogenin, Cytosporone B, Cytotrienin A, Cytotrienin I, Cytotrienin III, Cytotrienin IV, Cytoxazone, D-(+)-Pinitol, Dacinostat, Dactimicin, Dactylfungin A, Dactylfungin B, Dactylocycline A, Dactylocycline B, Dactylorhin B, DADMe-Immucillin-G, Dalfopristin mesilate, Dalvastatin, Damnacanthal, Damunacantal, Dapagliflozin, Daphnodorin A, Daphnodorin B, Daphnodorin C ((-)-enantiomer), Dapitant, Dapropterin dihydrochloride, Darbufelone, Darbufelone mesilate, Darunavir, Dasatinib, Daunomycin, Daunorubicin, Davidigenin, Decarestrictine C, Decarestrictine D, Decitabine, Decursinol, Decyl gallate, Deferiprone, Deforolimus, Dehydelone, Dehydrodidemnin B, Dehydrodolastatin-13, Dehydroepiandrosterone, Dehydroilludin M, Dehydrozingerone, Delaminomycin A, Delaminomycin B, Delaminomycin C, Delphinidin, Delphinidin chloride, Delphinidol, Deltacortisone, Deltadehydrocortisone, delta-Tocopherol glucoside, delta-Tocopheryl glucoside, Demethimmunomycin, Demethomycin, Demethylallosamidin, Demethylasterriquinone B-1, Denopamine, Denufosol tetrasodium, Deoxycoformycin, Deoxyenterocin, Deoxymulundocandin, Deoxynojirimycin, Deoxyspergualin hydrochloride, Deprodone propionate, Desacetylravidomycin N-Oxide, Desacetylvinblastinehydrazide, Desbutylhalofantrine hydrochloride, Desferri-salmycin A, Desferri-salmycin B, Desferri-salmycin C, Desferri-salmycin D, Desisobutyrylciclesonide, Deslorelin, Desmethyleleutherobin, Desogestrel, Desoxyepothilone B, Desoxyepothilone F, Desoxylaulimalide, Dexamethasone, Dexamethasone cipecilate, Dexamethasone palmitate, Dexamethasone sodium phosphate, Dexanabinol, Dexanabinone, Dexketoprofen choline, Dexsotalol, Dextrorphan, Dezocitidine, D-Fluviabactin, DHA-paclitaxel, Diadenosine tetraphosphate, Diazepinomicin, Diazoxide choline salt, Dicoumarol, Dictyostatin, Dictyostatin 1, Dicumarol, Didanosine, Didehydro-epsilon-viniferin, Didemnin X, Didemnin Y, Dideoxycytidine, Dideoxyinosine, Dieckol, Diepoxin-sigma, Difimicin, Digalactosyldiacylglycerol, Digoxin, Diheteropeptin, Dihydrexidine, Dihydro-alpha\-ergokryptine mesylate, Dihydrocalanolide A-(+), Dihydrocostatolide, Dihydroeponemycin, Dihydroergotamine mesylate, Dihydroheptaprenol, Dihydrohonokiol B, Dihydroraloxifene, Dihydrotestosterone, Dilevalol, Dilevalol hydrochloride, Dimelamol, Dimethyl-APD, Dimethylcurcumin, Dinaphine, Dinapsoline, Dinofan, Dinoxyline, Dioxolane T, Dioxolane thymine nucleoside, Diperamycin, Dipivefrine hydrochloride, Dipotassium gossypolate, Dipranol hydrochloride, Dipyridamole, Dipyridamole beta-cyclodextrin complex, Diquafosol tetrasodium, Dirithromycin, Discodermide, Discodermide acetate, Discodermolide, Disermolide, Disodium cromoglycate, Disodium cromproxate, Disodium lettusate, Disodium pamidronate, Disoprofol, Disorazol E1, Disorazole E1, Dobutamine hydrochloride, Dobutamine phosphate, Docetaxel, Docetaxol, Docosanol, Docosyl cidofovir, Dolastatin 13, Dopexamine, Dopexamine hydrochloride, Doramectin, Doranidazole, Doretinel, Doripenem, Dosmalfate, Doxefazepam, Doxercalciferol, Doxifluridine, Doxorubicin hydrochloride, Doxorubicin, morpholinyl, Doxycycline hyclate, Doxycycline hydrochloride ethanol hydrate, d-Pseudoephedrine hydrochloride, Dridocainide, Droxinavir, Duocarmycin B1, Duocarmycin B2, Duocarmycin C1, Duocarmycin C2, Dutomycin, Dynemicin C, Ecdysterone, Ecomustine, Ecopipam, Ecraprost, Ecteinascidin 722, Ecteinascidin 729, Ecteinascidin 736, Ecteinascidin 743, Ecteinascidin 745, Ecteinascidin 757, Ecteinascidin 770, Ecteinascidin 875, Edotreotide yttrium, Efepristin, Eflucimibe, Eflumast, Efonidipine hydrochloride ethanol, Eicosyl cidofovir, Elacytarabine, Elaidic acid-Cytarabine, Eldacimibe, Eldecalcitol, Eleutherobin, Eleutheroside B, Eliprodil, Elisapterosin B, Ellagic acid-4-gallate, Elliptoside A, Elliptoside E, Elocalcitol, Elomotecan hydrochloride, Eltanolone, Elvucitabine, Emakalim, Embeconazole, Embelic acid, Embelin, Emestrin C, Emodin, Emodol, Emoxyl, Emtricitabine, Enalkiren, Enazadrem, Enigmol, Enocitabine, Enofelast, Enpiperate, Enprostil, Entacapone, Entecavir, Enteric-Coated Mycophenolate Sodium, ent-Estriol, ent-Etorphine, Eperezolid, Epervudine, Ephdine, Epicochlioquinone A, Epidoxoform, Epidoxorubicin, Epigallocatechin gallate, Epigallocatechin-3-gallate, Epirubicin hydrochloride, Epispongiadiol, Eplivanserin, Eplivanserin mesilate, Epocarbazolin A, Epocarbazolin B, Epolactaene, Eponemycin, Epothilone A, Epothilone A N-oxide, Epothilone B N-oxide, Epothilone D, Epothilone E, Epoxomicin, Epoxyvibsanin B, Eptastatin sodium, Eptazocine hydrobromide, Erabulenol A, Erabulenol B, Erectumin A, Eribulin mesilate, Eribulin mesylate, Eritoran tetrasodium, Ersentilide, Ersentilide hydrochloride, Eryloside A, Erythritol, Erythrodiol, Erythromycin, Esculeoside A, Esmolol hydrochloride, Espatropate hydrate, Esperamicin A1, Esperatrucin, Estetrol, Estradiol, Estriol, Etalocib sodium, Etamsylate, Ethamsylate, Ethanolamine, Ethanolamine oleate, Ethchlorvynol, Ethinyl estradiol, Ethinylestradiol, Ethinyloestradiol, Ethylthio-DADMe-immucillin-A, Ethynylcytidine, Etidronate disodium, Etidronic acid disodium salt, Etiprednol dicloacetate, Etonogestrel, Etoposide, Etoposide phosphate disodium salt, Eugenodilol, Eugenosedin A, Euphodendroidin D, Eurotinone, Euxanthone, Evernimicin, Everolimus, Exatecan mesilate, Exifone, Factor P-Zyma, Fadolmidine hydrochloride, Faeriefungin A, Faeriefungin B, Falecalcitriol, Famotidine bismuth citrate, Faropenem daloxate, Faropenem medoxomil, Faropenem sodium, Favipiravir, Febuprol, Fenoldopam mesilate, Fenoterol hydrobromide, Fepradinol, Ferpifosate sodium, Ferulinolol, Fidaxomicin, Filibuvir, Fimbrigal P, Fingolimod hydrochloride, Finrozole, Flavalfate, Flavopiridol, Flocalcitriol, Flomoxef sodium, Floxuridine, Fluconazole, Fludarabine phosphate, Fludelone, Fludeoxyglucose (18F) , Fluindostainin sodium, Flumecinol, Flunisolide, Fluocinolide (formerly), Fluocinonide, Fluorodeoxyglucose (18F), Fluoroindolocarbazole A, Fluoroindolocarbazole B, Fluoroindolocarbazole C, Fluoroneplanocin A, Fluostatin A, Fluostatin B, Flupenthixol hydrochloride, Flupentixol hydrochloride, Fluphenazine hydrochloride, Flurithromycin, Fluticasone furoate, Fluticasone propionate, Flutropium bromide, Fluvastatin sodium, Fluvirucin B2, Foetidine 1, Foetidine 2, Folipastatin, Fondaparin sodium, Fondaparinux sodium, Formestane, Formobactin, Formosyn A, Forodesine hydrochloride, Forskolin, Fortimicin A, Fosaprepitant dimeglumine, Fosfomycin trometamol, Fosfomycin tromethamine, Fosopamine, Fosteabine sodium hydrate, Frederine, Fropenem sodium, Fucoxanthin, Fujianmycin B, Fujimycin, Fuladectin component A3, Fuladectin component A4, Fulvestrant, Fumagalone, Furaquinocin A, Furaquinocin B, Furopenem, Fusacandin A, Fusacandin B, Fuscoside B, Fusidate silver, Gaboxadol, Gabusectin methyl ester, Gadobutrol, Gadocoletic acid trisodium salt, Gadoteridol, Galactomycin I, Galactomycin II, Galamustine hydrochloride, Galarubicin hydrochloride, Galocitabine, Galparan, gamma-Hydroxybutyrate sodium, gamma-Mangostin, gamma-Tocotrienol, Ganciclovir, Ganciclovir elaidic acid, Ganciclovir monophosphate, Ganciclovir sodium, Ganglioside GM1, Ganirelix, Garomefrine hydrochloride, Garveatin E, Garveatin F, Gemcitabine, Gemcitabine elaidate, Gemeprost, Genaconazole, Genipin, Genistein-7-phosphate, Gestrinone, Gigantol, Gilvusmycin, Gimatecan, Gimeracil, Gimestat, Giracodazole, Girodazole, Girolline, Glaucocalyxin A, Glemanserin, Glenvastatin, Glidobactin PF-1, Glucarolactam potassium, Glucolanomycin, Glucolipsin A, Glucolipsin B, Glucopiericidinol A1, Glucopiericidinol A2, Glucosamine sulfate, Glufosfamide, Glycopyrrolate, Glycopyrronium bromide, Glycothiohexide alpha, Gomphostenin, Gomphostinin, Goodyeroside A, Goserelin, Granaticin B, Gusperimus hydrochloride, Gusperimus trihydrochloride, Gypsetin, Habekacin, Habekacin sulfate, Halistatin 1, Halistatin 2, Halistatin 3, Halobetasol propionate, Halofantrine hydrochloride, Halofuginone hydrobromide, Halometasone, Haloperidol, Halopredone acetate, Halovir A, Halovir B, Halovir C, Halovir D, Halovir E, Halxazone, Haperfonrin A, Haperforin B1, Haperforin F, Haperforine A, Haperforine B1, Haperforine F, Hatomamicin, Hatomarubigin A, Hatomarubigin B, Hatomarubigin C, Hatomarubigin D, Hattalin, Hayumicin B, Hayumicin C2, Hayumicin D, Hederacolchiside E, Heliquinomycin, Heptaminol AMP amidate, Hexadecyl cidofovir, Hexadecyloxypropyl-cidofovir, Hexafluorocalcitriol, Hidrosmin, Hidrosmina, Himastatin, Hispitolide D, Histrelin, Homorisedronate, Honokiol diepoxide, Honokiol diepoxide, Hyaluronan, Hyaluronate sodium, Hydrocortisone aceponate, Hydromorphone methiodide, Hydroxyakalone, Hydroxychloroquine sulfate, Hydroxymethylacylfulvene, Hydroxymycotrienin A, Hydroxyphoslactomycin B, Hymenistatin 1, Hyoscine, Hyoscine methobromide, Hypeptin, Hypericin, Hyperin, Hyperoside, Hypocholaride, Ibandronate sodium hydrate, Ibandronic acid monosodium salt monohydrate, Icariin, Idarubicin hydrochloride, Idebenone, Idremcinal, Ifenprodil, Ilatreotide, Iliparcil, Ilonidap, Imidacrine, Imidazoacridinone, Immucillin-H, Immunosine, Implitapide, Incyclinide, Indacaterol, Indanocine, Indinavir sulfate, Indomethacin trometamol, Indomethacin tromethamine, Indomethacin-Simvastatin, Ingenol mebutate, Inophyllum B, Inophyllum P, Inosine pranobex, Inosiplex, Inositol triphosphate, Intaxel (from Himalayan Yew), Integracide A, Integracide B, Integracin A, Integracin B, Integracin C, Integramycin, Integrastatin A, Integrastatin B, Intoplicine, Iobitridol, Iodochlorhydroxyquin, Iodorubidazone (p), Iofratol, Iolopride (123I) , Iomeprol, Iopamidol, Iopentol, Iopromide, Iotriside, Iotrol, Iotrolan, Ioversol, Ioxilan, Ioxipride, Ipratropium bromide, Iralukast sodium, Irciniastatin A, Irciniastatin B, Irinotecan hydrochloride, Irofulven, Isalmadol, Isatoribine, Isavuconazole, Isavuconazonium chloride hydrochloride, Iseganan hydrochloride, Isepamicin sulfate, Isobavachalcone, Isodoxorubicin, Isoeleutherobin A, Isofloxythepin, Isohomohalichondrin B, Isoliquiritigenin, Isomolpan hydrochloride, Isoquine, iso-Simvastatin-6-one, Isosorbide 5-mononitrate, Isospongiadiol, Isovanihuperzine A, Isoxazoledehydelone, Isoxazolefludelone, Itavastatin calcium, Itrocinonide, Iturelix, Ixabepilone, Jadomycin B, Janthinomycin A, Janthinomycin B, Janthinomycin C, Jaspamide, Jaspine B, Jasplakinolide, Jorumycin, Kadsuphilin C, Kahalalide F, Kampanol A, Kampanol B, Kanamycin, Kanamycin A, Kansuinin B, Karalicin, Karnamicin B1, Katanosin A, Katanosin B, Katanosin B, Kehokorin D, Kehokorin E, Keoxifene hydrochloride, Kifunensine, Kigamicin A, Kigamicin B, Kigamicin D, Kigamicin E, Kigamicinone, Kinsenoside, Klainetin A, Klainetin B, Kobifuranone B, Kobiin, Kodaistatin A, Kodaistatin C, Kodaistatin D, Korupensamine A, Korupensamine B, Korupensamine C, Korupensamine D, Kosinostatin, Kukoline, Kushenol F, Kushnol F, Kynostatin-227, Kynostatin-272, Labedipinedilol A, Labedipinedilol B, Labetalol hydrochloride, Lactonamycin, Lactosylphenyl trolox, Ladakamycin, Ladirubicin, Lagatide, Laherradurin, Lamellarin alpha 20-sulfate sodium salt, Lamivudine, Landiolol, Larotaxel dihydrate, Lasinavir, Lasofoxifene, Lasonolide A, Latamoxef sodium, Latanoprost, Latrunculin S, Lavanduquinocin, L-Dopa methyl ester hydrochloride, L-Dopamide, Lecirelin, Ledazerol, Leinamycin, Leptocillin, Leptofuranin A, Leptofuranin B, Lersivirine, Lestaurtinib, Leurubicin, Leustroducsin A, Leustroducsin B, Leustroducsin C, Leustroducsin H, Levalbuterol hydrochloride, Levdropropizine, Levobetaxolol hydrochloride, Levobunolol hydrochloride, Levodopa methyl ester hydrochloride, Levodropropizine, Levonebivolol, Levonorgestrel, Levonorgestrel 3-oxime, Levosalbutamol hydrochloride, Lexacalcitol, L-Fluviabactin, L-Fluvibactin, L-Fluvibactine, L-Histidinol, Liblomycin, Lificiguat, Linderol A, Linopristin, Linopristine, Lipiarmycin, Lipiarmycin A3, Lipiarmycin B3, Lipiarmycin B4, Lipo-isocarbacyclin methyl ester, Liquiritin apioside, Lisofylline, Lithospermic acid B magnesium salt, l-Nebivolol, Lobatamide C, Lobatamide F, Lobophorin A, Lobophorin B, Lobucavir, Lobucavir, Lodenafil, Lodenosine, Lonaprisan, Loperamide hydrochloride, Lopinavir, Lorazepam, Lornoxicam, Losartan, Losartan potassium, Losigamone, Loteprednol etabonate, Lovastatin, Loxoribine, L-threitol ceramide, L-threo-C6-pyridinium-ceramide-bromide, Lubeluzole, Lumefantrine, Luminacin D, Lurtotecan, Luteolin, Lycopersicin, Lysobactin, Mabuterol hydrochloride, Macquarimycin B, Macrocarpin A, Macrolactine M, Madecassoside, Madindoline A, Madindoline B, Magnesium lithospermate B, Magnesium salvianolate B, Magnesium tanshinoate B, Malonoben, Manifaxine hydrochloride, Manitimus, Mannopeptimycin alpha, Mannopeptimycin beta, Mannopeptimycin delta, Mannopeptimycin epsilon, Manoalide, Manumycin A, Manumycin B, Manumycin C, Manumycin E, Manumycin F, Manumycin G, Manzamine A, Manzamine D, Manzamine E, Manzamine F, Maribavir, Marmelin, Masoprocol, Maxacalcitol, Mazokalim, Medicarpin, Medigoxin, Mefloquine hydrochloride, Megovalicin A, Megovalicin B, Megovalicin C, Megovalicin D, Megovalicin G, Megovalicin H, Melevodopa hydrochloride, Mellein, Meloxicam, Meluadrine, Mepenzolate bromide, Mepindolol sulfate, Mepindolol transdermal patch, Meptazinol hydrochloride, Metaproterenol sulfate, Metaraminol, Meterelin, Methoxatone, Methscopolamine bromide, Methyl bestatin, Methylnaltrexone bromide, Methylprednisolone, Methylprednisolone suleptanate, Methyltestosterone, Methypranolol, Metildigoxin, Metipranolol, Metrifonate, Metriphonate, Metronidazole, Mevinolin, Micacocidin A, Micafungin sodium, Michellamine B, Michigazone, Microcolin A, Micronomicin sulfate, Midecamycin acetate, Mideplanin, Midoriamin, Mifepristone, Miglitol, Miglustat, Milataxel, Milbemycin alpha-9, Mimopezil, Minocycline hydrochloride, Minodronate, Minodronic acid, Miokamycin, Miporamicin, Mipragoside, Miproxifene, Mirabegron, Mirodenafil hydrochloride, Misakinolide, Misoprostol, Mitoxantrone hydrochloride, Mitozantrone hydrochloride, Mivazerol, Mivobulin isethionate, Mizoribine, Modecainide, Modithromycin, Moenomycin A chloride bismuth salt, Mollugin, Mometasone furoate, Monakolin K, Monlicin A, Monoethanolamine oleate, Monogalactosyldiacylglycerol, Monophosphoryl lipid A, Montelukast sodium, Moranolin, Moxalactam disodium, Moxidectin, Mozenavir mesilate, Multiforisin A, Muramyl dipeptide C, Mycalamide A, Mycalamide B, Mycinamicin II, Mycolactone A, Mycolactone B, Mycophenolate mofetil, Mycophenolate sodium, Mycophenolic acid sodium salt, Myrciacitrin I, Myrciacitrin II, Myrciaphenone B, Myrocin C, Mytolbilin, Mytolbilin acid methyl ester, Mytolbilinol, Myxovirescin A1, Myxovirescin B, N4-Hexadecyl-dC-AZT, N-9-Oxadecyl-6-methyl-DGJ, Naamidine A, Nabilone, N-Acetylcolchinol, N-Acetylesperamycin A1, N-Acetylesperamycin A1b, N-Acetylesperamycin A2, Nadolol, Nafocare B1, Naftopidil, Nafuredin-gamma, Nalfurafine hydrochloride, Nalmefene, Nalmetrene, Naloxone hydrochloride, Naltrexone hydrochloride, Naltrindole, Namitecan, Nardeterol, Naroparcil, Navelbine, Navuridine, N-Butyl-deoxynojirimycin, NCP-tazopsine, N-Cyclopentyl-tazopsine, Nebivolol, Nectrisine, Nelarabine, Neldazosin, Nelfinavir mesilate, Nelivaptan, Nelzarabine, Nemorubicin, Neo-acridine, Neocimicigenoside B, Neolaulimalide, Neomycin B-arginine conjugate, Neomycin B-hexaarginine conjugate, Neomycin-acridine, Nepadutant, Neparensinol A, Neparensinol B, Neparensinol C, Neridronate, Neridronic acid, Neristatin 1, Nesbuvir, Netilmicin sulfate, Netivudine, Ngercheumicin A, Ngercheumicin B, N-hexacosanol, Nicanartine, Nifekalant hydrochloride, Nimesulide choline salt, Nipradilol, Nipradolol, Nisvastatin, Nitecapone, Nitrofen, Nitrophene, Nitropravastatin, N-Methylhydromorphonium iodide, N-Nonyl-deoxygalactojirimycin, Nocardione A, Nocathiacin I, Nocathiacin II, Nocathiacin III, Nocathiacin IV, NO-hydrocortisone, NO-Mesalamine, NO-Pravastatin, Noraristeromycin, Nordamnacanthal, Nordamunacantal, Nordihydroguaiaretic acid, Norelgestromin, Norkurarinone, Normethyljiadifenin, Nortopixantrone hydrochloride, Nostocyclopeptide M1, Nothramicin, NO-Ursodeoxycholic acid, N-Retinoyl-D-glucosamine, N-tert butyl isoquine, Nubiotic 2, Nutlin-2, Obelmycin H, Oberadilol, Ochracin, Ochromycinone, Ocimumoside A, Ocimumoside B, Octreother, Octyl gallate, O-Demethylmurrayafoline A, Oenothein B, Okicenone, Olcegepant, Olmesartan medoxomil, Olpadronic acid sodium salt, Olsalazine sodium, Omaciclovir, Ombrabulin, Ombrabulin hydrochloride, Onjixanthone I, Onjixanthone II, Oolonghomobisflavan A, Oolonghomobisflavan C, Opipramol hydrochloride, Orciprenaline sulphate, Ornoprostil, Orthosomycin A, Orthosomycin B, Orthosomycin C, Orthosomycin E, Orthosomycin F, Orthosomycin G, Orthosomycin H, Ospemifene, Osutidine, OTAC, Ovalicin, Ovalicin A, Oxandrolone, Oxaspirol A, Oxaspirol B, Oxazepam, Oxazofurin, Oxeclosporin, Oximidine III, Oxiracetam, Oxitropium bromide, Oxolide, Oxprenolol hydrochloride, Oxybutynin chloride, Oxybutynin hydrochloride, Oxydevit, Oxymetazoline hydrochloride, Oxymethacyl, Oxymorphazole dihydrochloride, Oxyphenarsine, Ozarelix, Pachastrissamine, Pachymedusa dacnicolor Tryptophyllin-1, Paciforgine, Paclitaxel, Paecilaminol, Paeciloquinine A, Pafenolol, Palau'amine, Palinavir, Paliperidone, Palosuran sulfate, Pamapimod, Pamaqueside, Pamidronate sodium, Panamesine hydrochloride, Pancratistatin disodium phosphate, Pancratistatin-3,4-cyclic phosphate sodium salt, Pannorin, Pantethine, Papuamide A, Papuamide B, Papuamide C, Papuamide D, Papyracillic acid, Paquinimod, Paracalcin, Paracetamol, Paraherquamide G, Paricalcitol, Paromomycin, Paroxetine ascorbate, Parthenicin, Parthenin, Parvisporin B, Patellazole A, Patellazole B, Patellazole C, Peloruside A, Penbutolol sulfate, Penciclovir, Penicillide, Pentamidine isethionate, Pentazocine hydrochloride, Pentostatin, Peplomycin, Pepluanin A, Pepticinnamin E, Periciazine, Pericyazine, Perillyl alcohol, Perphenazine, Persin, Petrosaspongiolide M, Phaffiaol, Phakellistatin 7, Phakellistatin 8, Phakellistatin 9, Phaseolinone, Phenochalasin B, Phenprocoumon, Phentolamine mesilate, Philinopside A, Phlorofucofuroeckol, Phlorofucofuroeckol A, Phomactin A, Phomactin B, Phomactin F, Phomactin G, Phomopsichalasin, Phorboxazole A, Phorboxazole B, Phospholine, Phosphostim, Piceatannol, Pidobenzone, Pidorubicin, Pimecrolimus, Pimilprost, Pindolol, Pinitol, Pinocembrin, Pipalamycin, Pipendoxifene, Pipenzolate bromide, Pipothiazine, Pipotiazine, Pirarubicin, Pirbuterol hydrochloride, Pirmenol hydrochloride, Pirodomast, Pironetin, Piroxicam, Pitavastatin calcium, Pittsburgh Compound B, Pladienolide A, Pladienolide C, Pladienolide D, Pladienolide E, Plantagoside, Plaunotol, Plitidepsin, Pluraflavin A, Pluraflavin B, Pneumocandin A0, Pneumocandin B0, Pneumocandin B0 2-phosphate, Pneumocandin D0, Podofilox, Podophyllotoxin, Poldine methylsulfate, Poldine methylsulphate, Poldine metilsulfate, Polyestradiol phosphate, Polyketomycin, Polymer bound human leukocyte elastase inhibitor, Popolohuanone E, Posaconazole, Posizolid, Potassium embelate, Prasterone, Pravastatin sodium, Prednicarbate, Prednisolone, Prednisolone acetate, Prednisolone farnesylate, Prednisone, Pregnanolone, Preussin, Prinaberel, Prinomide tromethamine, Prisotinol, Pristinamycin IA, Pristinamycin IB, Pristinamycin IIA, Probucol, Procaterol hydrochloride hemihydrate, Procyclidine hydrochloride, Propafenone hydrochloride, Propericiazine, Propofol, Propranolol hydrochloride, Propyl gallate, Prostanit, Prostratin, Protegrin IB-367, Protocatechuic aldehyde, Proxodolol, Pseudoephedrine hydrochloride, Pseudohypericin, Psymberin, Ptidepsin, Purpuromycin, Purvalanol A, Pyridavone, Pyrindamycin A, Pyrindamycin B, Pyripyropene A, Pyripyropene B, Pyripyropene C, Pyripyropene D, Pyrrocidine A, Pyrrocidine B, Quartromicin A1, Quartromicin A2, Quartromicin A3, Quartromicin D1, Quartromicin D2, Quartromicin D3, Quercetin 3-galactoside, Quercetin 3-O-beta-D-galactopyranoside, Quercetin-3-O-methyl ether, Quercinitol, Quinagolide hydrochloride, Quinidine, Quinobene, Quinoxapeptin C, Quinupristin mesilate, rac-Apogossypolone, rac-Tolterodine, Radolmidine hydrochloride, Raloxifene hydrochloride, Raluridine, Rameswaralide, Ramoplanin A'1, Ramoplanin A'2, Ramoplanin A'3, Ramorelix, Rancinamycin IV, Ranitidine bismuth citrate, Ranitidine Bismutrex, Ranitidine zinc citrate, Ranolazine, Rapamycin, Ravidomycin N-Oxide, Ravuconazole, Rawsonol, Reblastatin, Relcovaptan, Remikiren mesilate, Remiprostol, Repandiol, Reproterol hydrochloride, Resobene, Resorthiomycin, Resveratrol, Retapamulin, Retaspimycin hydrochloride, Revatropate, Rhodiocyanoside A, Rhodiocyanoside B, Rhodostreptomycin A, Ribamidine hydrochloride, Ribavirin, Ribavirin eicosenate cis, Ribavirin eicosenate trans, Ribavirin elaidate, Rifabutin, Rifalazil, Rifamexil, Rifampicin, Rifampin, Rifapentine, Rifaximin, Rilmakalim hemihydrate, Rimexolone, Riodoxol, Risedronate sodium, Ritipenem acoxil, Ritonavir, Rivastatin, Rivenprost, Rocuronium bromide, Rofleponide, Rofleponide palmitate, Rohitukine, Rolliniastatin 1, Ronoprost, Roquinimex, Rosaprostol sodium, Roscovitine, Roselipin 1A, Roselipin 1B, Roselipin 2B, Rostafuroxine, Rosuvastatin calcium, Rosuvastatin sodium, Rotigaptide, Rotigotine, Roxatidine bismuth citrate, Roxindole mesilate, Rubiginone A1, Rubiginone A2, Rubiginone B1, Rubiginone C1, Rubitecan, Ruboxyl, Rufigallol, Rugatocenone B, Russuphelin A, Sabarubicin hydrochloride, Safingol, Sagamacin, Saintopin, Saintopin E, Saishin N, Sakyomicin A, Sakyomicin E, Salbostatin, Salbutamol nitrate, Salbutamol sulfate, Salcaprozic acid sodium salt, Salicylihalamide A, Salicylihalamide B, Salinamide A, Salinosporamide A, Saliphenylhalamide, Salmaterol, Salmeterol, Samaderine X, Sanfetrinem cilexetil, Sanfetrinem sodium, Sanglifehrin A, Sanglifehrin B, Sanglifehrin C, Sanglifehrin D, Sanilvudine, Sapacitabine, Sapropterin dihydrochloride, Saptomycin D, Saquinavir, Saquinavir mesilate, Sarcophytol A, Sarcophytol B, Saricandin, Satoribine, Saxagliptin, Sazetidine-A, Schisandrin, Schisandrol A, Schizandrin, Scopolamine, Scyllitol, Scyllo-Inositol, Scyphostatin, Sebacoyl dinalbuphine ester, Secalciferol, Secobatzelline A, Secobatzelline B, Seglitide, Selamectin, Selodenoson, Semagacestat, Semorphone hydrochloride, Seocalcitol, Seprilose, Sergliflozin etabonate, Setamycin, Setazindol, Sevelamer carbonate, Shishijimicin A, Shishijimicin B, Shishijimicin C, Sialosylcholesterol-alpha sodium salt, Sialosylcholesterol-beta sodium salt, Sibanomicin, Sibenadet hydrochloride, Silodosin, Siltenzepine, Silychristin, Simotaxel , Simvastatin, Sinomenine, Sirolimus, Sitostanol ascorbyl phosphate, Sivifene, Siwenmycin, Smirinol, Smyrinol, Socorromycin, Sodium azodisalicylate, Sodium cromoglycate, Sodium oxybate, Solidagenon, Solidagenone, Solpecainol hydrochloride, Sonedenoson, Sophoraflavanone G, Soraprazan, Sorivudine, Sotalol hydrochloride, Sparoxomycin A1, Sparoxomycin A2, Sperabillin A, Sperabillin B, Sperabillin C, Sperabillin D, Spinosulfate A, Spinosulfate B, Spiralizone B, Spirocardin A, Spirocardin B, Spiruchostatin A, Spiruchostatin B, Spisulosine, Spisulosine 285, Spongiadiol, Spongistatin 1, Spongistatin 2, Spongistatin 3, Spongistatin 4, Spongistatin 5, Spongistatin 6, Spongistatin 7, Spongistatin 8, Spongistatin 9, Sporeamicin A, Sporeamicin B, Stachybotrin C, Stachybotrydial, Stavudine, Stelleramacrin A, Stelleramacrin B, Sterenin C, Streptomycin, Streptopyrrole, Styloguanidine, Sufotidine bismuth citrate, Sugammadex sodium, Sulfinosine, Sulfircin C, Sulopenem etzadroxil, Sulphazocine, Sulphoquinovosyldiacylglycerol, Sulprostone, Super-Leu-Dox, Symbioimine, Synadenol, Synvinolin, Syriacusin A, Syriacusin B, Syriacusin C, Syringin, Syzygiol, Tacalcitol, Tacapenem pivoxil, Taccalonolide E, Tacrolimus, Tafluprost, Taiwanhomoflavone A, Takanawaene A, Takanawaene B, Takanawaene C, Talnetant, Tamandarin A, Tamolarizine hydrochloride, TAP-doxorubicin, Tapentadol hydrochloride, Taramanon A, Taribavirin hydrochloride, Tasquinimod, Taurohyodeoxycholic acid, Tauroiodeoxycholic acid, Tautomycin, Taxuyunnanine, Tazofelone, Tazopsine, Tebipenem cilexetyl, Tebipenem pivoxil, Tebufelone, Tecadenoson, Technetium (99mTc) depreotide, Technetium Tc 99m depreotide, Teicoplanin-A2-1, Teicoplanin-A2-2, Teicoplanin-A2-3, Teicoplanin-A2-3, Teicoplanin-A2-5, Telbivudine, Telinavir, Temazepam, Temiverine, Temiverine hydrochloride hydrate, Temoporfin, Tempol, Temserolimus, Temsirolimus, Tenidap, Teniposide, Tenoxicam, Tenuifoliside A, Tenuifoliside B, Tenuifoliside C, Tenuifoliside D, Terasin, Terbutaline sulfate, Terfenadine, Teriflunomide, Terlakiren, Terlipressin, Ternatin, Terprenin, Terreulactone A, Terreulactone C, Terreulactone D, Tertatolol hydrochloride, Tesetaxel, Tetrabromostyloguanidine, Tetracosyl cidofovir, Tetrahydrobiopterin, Tetrahydrocortisol, Tetrahydrocurcumin, Tetrahydroechinocandin B, Tetrahydroswertianolin, Tetrahydroxyquinone, Tetrazolast meglumine, Tetromycin A, Tetromycin B, Tetroquinone, Texenomycin A, Tezacitabine, Tezosentan, Tezosentan disodium, Thenorphine, Theopederin D, Theoperidin E, Theophylline rutoside, Theprubicin, Thiamet-G, Thiamphenicol, Thiarubrine E, Thiarubrine F, Thiarubrine G, Thiarubrine H, Thiazinotrienomycin B, Thiazinotrienomycin F, Thiazinotrienomycin G, Thienorphine, Thiocoraline, Thiocoraline A, Thiocoraline NF, Thiofedrine, Thiofoscarnet, Thiomarinol, Thiomarinol B, Thiomarinol E, Thioviridamide, Threitol ceramide, Thymallene, Thymectacin, Tiacumicin B, Tibolone, Tidembersat, Tienoxolol hydrochloride, Tigecycline, Tigilcycline, Tigogenin cellobioside (beta), Tilisolol hydrochloride, Timolol hemihydrate, Tiotropium bromide, Tiplimotide, Tipranavir, Tiqueside, Tisocalcitate, Tixocortol buryrate propionate, Toborinone, Tocotrienol, Tokaramide A, Tolcapone, Toloxatone, Tolytoxin, Tomatine, Tomeglovir, Tonabersat, Topitriol, Topixantrone hydrochloride, Topotecan hydrochloride, Topsentin, Topsentine B1, Torcitabine, Torezolid, Toripristone, Tosagestin, Tosedostat, Trabectedin, Tradecamide, Tramadol hydrochloride, Tramadol N-oxide, trans-Resveratrol, Trantinterol hydrochloride, Traxoprodil, Trecadrine, Treprostinil, Trewiasine, Triamcinolone acetonide, Triamcinolone hexacetonide, Tribavirin, Trichlorfon, Trichodimerol, Trichostatin D, Triciferol, Triciribine, Triciribine phosphate, Triciribine-5'-monophosphate, Trifluridine, Trihexyphenidyl hydrochloride, Trilostane, Trimazosin hydrochloride, Trimegestone, Trimexolone, Trimidox, Triphendiol, Tripterifordin, Tripterinin, Triptolide, Troglitazone, Troxacitabine, Troxerutin, Tsukubamycin A, Tubastrine ((+)-enantiomer), Tuberactomycin B, Tubingensin B, Tulathromycin A, Tulathromycin B, Tulobuterol hydrochloride, Turbostatin 2, Turbostatin 3, Turbostatin 4, Tyropeptin A10, Tyropeptin A6, Tyropeptin A9, Tyrphostin 47, Tyrphostin A9, Tyrphostin AG-213, Ubenimex methyl ester, Ukrain, Ulobetasol propionate, Uncialamycin, Uniprost, Unit dose budesonide, Unoprostone isopropyl ester, Ustilipid A, Ustilipid B, Ustilipid C, Uvalol, Uvaol, Valnemulin, Valrubicin, Vancomycin hydrochloride, Vancoresmycin, Vaninolol, Variapeptin, Vastribil, V-Echinocandin, Veinamitol, Venlafaxine N-oxide, Venorphin, Vermisporin, Vernakalant hydrochloride, Verticillatine, Vexibinol, Vialinin B, Vicenistatin, Vildagliptin, Vincristine sulfate, Vindesine, Vinflunine, Vinfosiltine sulfate, Viniferifuran, Vinleucinol, Vinorelbine, Vinxaltine sulfate, Viramidine hydrochloride, Viranamycin-B, Virginiamycin M1, Vitamin-P4, Voclosporin, Voglibose, Volinanserin, Voriconazole, Wangzaozine B, Wiedendiol A, Wiedendiol B, Woodorien, Wuweizichun A, Xanthoangelol E, Xanthomegnin, Xenovulene A, Xipamide, Xydiphone (K,Na salt), Xylocydine, Yatakemycin, Yohimbine, Yttrium-90 edotreotide, Yunaconitine, Zahavin B, Zalcitabine, Zampanolide, Zankiren, Zanoterone, Zelandopam hydrochloride, Z-Eleutherobin, Zidovudine, Zilascorb (2H), Zilpaterol, Zorubicin hydrochloride, Zotarolimus, Zoticasone propionate, and Zuclopenthixol hydrochloride.

Another object of the present invention is a pharmaceutical composition comprising a prodrug of the present invention or a pharmaceutical salt thereof together with a pharmaceutically acceptable excipient.

Yet another object of the present invention is a prodrug or a pharmaceutically acceptable salt thereof of the present invention or a pharmaceutical composition of the present invention for use as a medicament.

Yet another object of the present invention is a prodrug or a pharmaceutically acceptable salt thereof of the present invention or a pharmaceutical composition of the present invention for use in the treatment, control, delay or prevention of one or more conditions in a mammalian patient

### Materials, Methods and Analytics:

### Chemicals:

Chemicals were purchased from Sigma Aldrich GmbH, Taufkirchen, Germany, if not stated otherwise. 6-(S-Tritylmercapto)hexanoic acid was purchased from Polypeptide, Strasbourg, France. Cis-cyclohexanedicarboxylic anhydride was purchased from Alfa Aesar GmbH & Co KG, Karlsruhe, Germany. Treprostinil acid was purchased from Chirogate International Inc. Yangmei, Taiwan. 2-Chlorotrityl chloride resin (1%, Novabiochem^{®} DVB) was obtained from Merck Biosciences GmbH, Germany. 6-(S-Tritylsulfanyl)-hexaneamine was synthesized according to WO-A 2009/133137). PEGs used in this work were acquired from NOF Europe N.V., Grobbendonk, Belgium.

### Product purification

Normal phase purification was performed on a Biotage "Isolera one" purification system Biotage AB, Sweden. Biotage KP-Sil silica cartridges. Gradients of Heptane/Ethylacetate or Dichloromethane/Methanol were used. Products were detected and collected at 254 and 280nm. For preparative RP-HPLC, a Waters 600 controller and a 2487 Dual Absorbance Detector was used equipped with a Waters XBridge^{™} BEH300 Prep C18 5 µm, 150 x 10 mm, flow rate 6 ml/min, or Waters XBridge^{™} BEH300 Prep C18 10 µm, 150 x 30 mm, flow rate 40 ml/min. Gradients of eluents A (water containing 0.05 % TFA v/v or 0.01 % HCl v/v) and B (acetonitrile containing 0.05 % TFA v/v or 0.01 % HCl v/v) were used.

HPLC fractions containing product were pooled and lyophilized if not stated otherwise.

### LC/MS Analytics

RP-HPLC/ESI-MS was performed on a Waters Acquity UPLC with an Acquity PDA detector coupled to a Thermo LTQ Orbitrap Discovery high resolution/high accuracy mass spectrometer equipped with a Waters ACQUITY UPLC BEH300 C18 RP column (2.1 x 50 mm, 300 Å, 1.7 µm, flow: 0.25 mL/min; solvent A: UP-H₂0 + 0.04% TFA, solvent B: UP-Acetonitrile + 0.05 % TFA.

### Example 1:

### Synthesis of building block 1

Building block 1 was synthesized according to the following scheme:

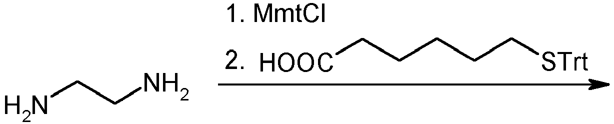

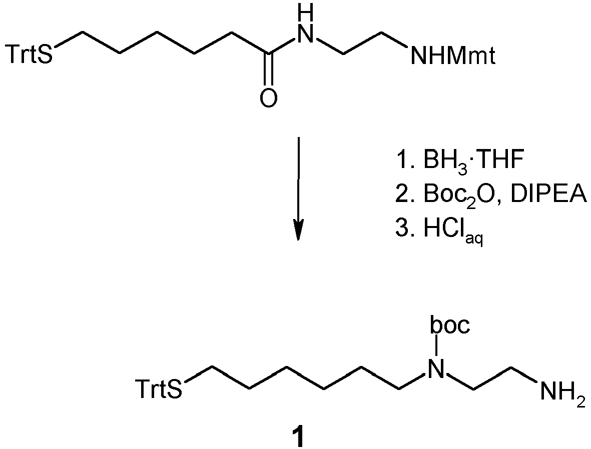

Mmt-chloride (3 g, 9.71 mmol) was dissolved in DCM (20 mL) and added dropwise to a solution of ethylenediamine (6.5 mL, 97.1 mmol) in DCM (20 mL). After two hours the solution was poured into diethyl ether (300 mL) and washed three times with 30/1 (v/v) brine/0.1 M NaOH solution (50 ml each) and once with brine (50 mL). The organic phase was dried over Na₂SO₄ and volatiles were removed under reduced pressure. Mmt-protected amine (3.18 g, 9.56 mmol) was used in the next step without further purification.

The Mmt-protected amine (3.18 g, 9.56 mmol) was dissolved in anhydrous DCM (30 mL). 6-(S-Tritylmercapto)hexanoic acid (4.48 g, 11.47 mmol), PyBOP ( 5.96 g, 11.47 mmol) and DIPEA (5.0 mL, 28.68 mmol) were added and the mixture was agitated for 30 min at RT. The solution was diluted with diethyl ether (250 mL) and washed three times with 30/1 (v/v) brine/0.1 M NaOH solution (50 mL each) and once with brine (50 mL). The organic phase was dried over Na₂SO₄ and volatiles were removed under reduced pressure. Amide was purified by flash chromatography eluting with heptane/ethyl acetate containing 0.02 % (v/v) diethylmethylamine.

Yield: 5.69 g (8.07 mmol).

MS: m/z 705.4 = [M+H]⁺ (MW = 705.0).

Amide (3.19 g, 4.53 mmol) was dissolved in anhydrous THF (50 mL) and BH₃•THF (1 M solution, 8.5 mL, 8.5 mmol) was added. Solution was stirred for 16 h at RT. Further BH₃•THF (1 M solution, 14 mL, 14 mmol) was added and stirred for further 16 h at RT. The reaction was quenched by addition of methanol (8.5 mL). N,N-dimethyl-ethylenediamine (3 mL, 27.2 mmol) was added, the solution was heated to reflux and stirred for 3 h. Reaction mixture was allowed to cool down to RT and was then diluted with ethyl acetate (300 mL), washed with saturated aqueous Na₂CO₃ solution (2 x 100 mL) and saturated aqueous NaHCO₃ solution (2 x 100 mL). The organic phase was dried over Na₂SO₄ and volatiles were removed under reduced pressure to obtain crude amine intermediate (3.22 g).

The amine intermediate (3.22 g) was dissolved in DCM (5 mL). Boc₂O (2.97 g, 13.69 mmol) dissolved in DCM (5 mL) and DIPEA (3.95 mL, 22.65 mmol) were added and the mixture was agitated at RT for 30 min. Boc- and Mmt-protected intermediate was purified by flash chromatography.

Yield: 3.00 g (3.79 mmol).

MS: m/z 791.4 = [M+H]⁺, 519.3 = [M-Mmt+H]⁺ (MW calculated = 791.1).

0.4 M aqueous HCl (48 mL) was added to a solution of the Boc- and Mmt-protected intermediate in acetonitrile (45 mL). The mixture was diluted with acetonitrile (10 mL) and stirred for 1 h at RT. Subsequently, the pH value of the reaction mixture was adjusted to 5.5 by addition of an aqueous 5 M NaOH solution. Acetonitrile was removed under reduced pressure and the aqueous solution was extracted with DCM (4 x 100 mL). The combined organic phases were dried over Na₂SO₄ and volatiles were removed under reduced pressure. Crude amine 1 was used without further purification.

Yield: 2.52 g (3.19 mmol). A MW of 791.1 g/mol of crude amine 1 was assumed

MS: m/z 519.3 = [M+H]⁺ (MW calculated = 519.8 g/mol).

### Example 2:

### Synthesis of linker building blocks 2a, 2b, and 2c

Linker building block **2a** was synthesized according to the following scheme:

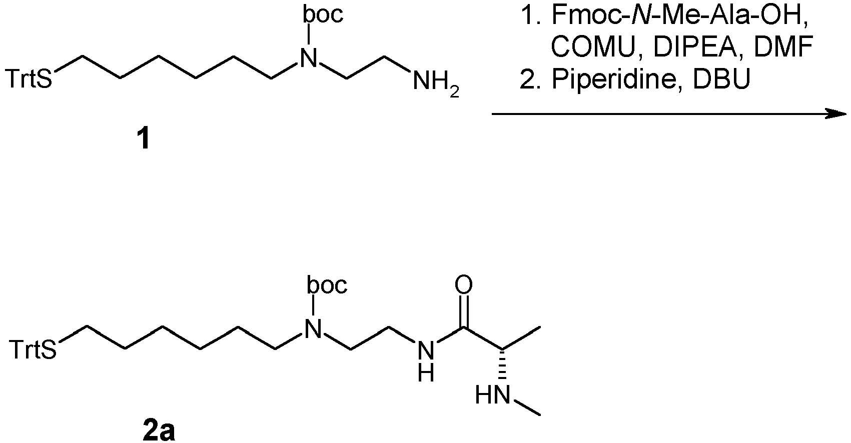

Amine 1 (503 mg, 0.635 mmol, assuming a MW of 791.1 g/mol of crude **1** ) was dissolved in 4 mL DMF (anhydrous, mol. sieve). Fmoc-*N*-Me-Ala-OH (310 mg, 0.953 mmol), COMU (408 mg, 0.953 mmol) and DIPEA (332 µl, 1.906 mmol) were added and the reaction was allowed to stir for 3 h at RT. 150 µl piperidine and 150 µl DBU were added to the mixture and stirring was continued for further 60 min. 400 µl acetic acid were added and product was purified by HPLC. HPLC fractions containing product **2a** were neutralized with a saturated aqueous NaHCO₃ solution and extracted twice with DCM. Combined organic phases were dried over Na₂SO₄ and volatiles were removed under reduced pressure.

Yield: 203 mg (0.336 mmol).

MS: m/z 604.1 = [M+H]⁺ (MW calculated = 603.9 g/mol).

### Linker building block 2b

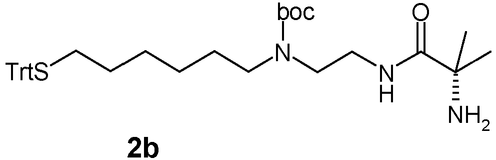

Linker building block **2b** was synthesized as described for **2a** except that Fmoc-Aib-OH was used instead of Fmoc-*N*-Me-Ala-OH.

Yield: 95 mg (0.161 mmol).

MS: m/z 604.2 = [M+H]⁺ (MW calculated = 603.9 g/mol).

### Linker building block 2c

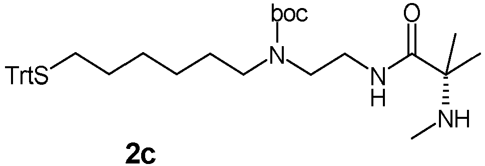

Linker building block **2c** was synthesized as described for **2a** except that Fmoc-*N*-Me-Aib-OH was used instead of Fmoc-*N*-Me-Ala-OH.

Yield: 149 mg (0.241 mmol).

MS: m/z 619.0 = [M+H]⁺ (MW calculated = 617.9 g/mol).

### Example 3:

### Synthesis of treprostinil-linker thiols 3a, 3b, 3c, 3d, 3e and 3f

Treprostinil-linker thiols **3a/3b** were synthesized according to the following scheme:

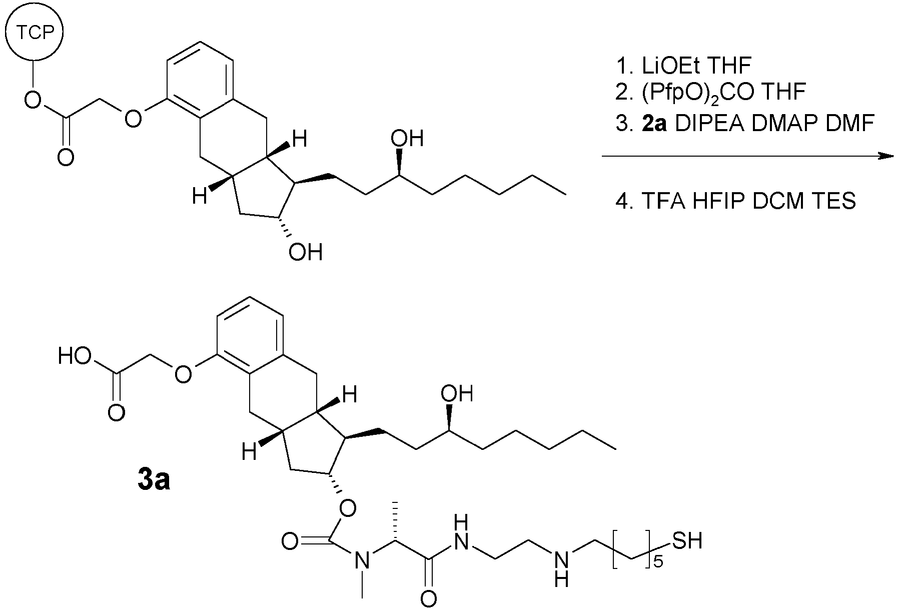

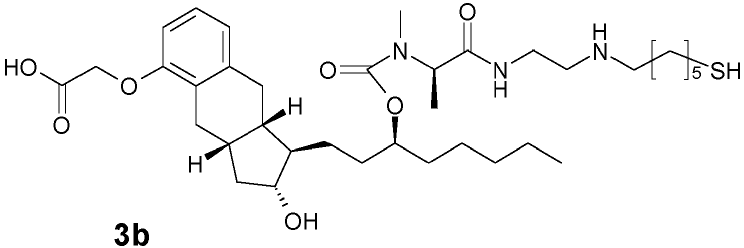

A 10 mL single use syringe reactor equipped with a PE frit was loaded with 2-chlorotrityl chloride (TCP) resin (153 mg, loading 1.22 mmol/g, 0.186 mmol). A solution of treprostinil (54 mg, 0.138 mmol) and DIPEA (60 µl, 0.346 mmol) in DCM (anhydrous, mol. sieve) was drawn into the reactor. Reactor was agitated for 2 h at RT. 200 µl methanol were added and reactor was agitated for further 10 min. Solution was dispelled and resin was washed with DCM (5x), DMF (5x) and DCM (10x). Resin was dried under vacuum (1 mbar). Based on weight, a treprostinil loading of 0.72 mmol/g TCP resin was obtained.

900 µl THF (anhydrous, mol. sieve) and 300 µl of a 1 M LiOEt solution in THF (300 µmol) were drawn to 30 mg treprostinil loaded TCP resin (21.6 µmol) in a single use 2 mL syringe reactor equipped with a PE frit. Reactor was agitated for 40 min at RT. Solution was dispelled and resin was washed with THF (2x). A solution of bis(pentafluorophenyl)carbonate (100mg, 254 µmol) in 1 mL THF was drawn into the syringe which was agitated for 90 min at RT. Solution was dispelled and resin was washed with THF (5x) and DMF (5x). A solution of linker building block **2a** (50 mg, 83 µmol), DIPEA (50 µl, 287 µmol) and DMAP (1 mg, 8 µmol) in 300 µl DMF (anhydrous, mol. sieve) was drawn into the syringe. Syringe was agitated for 3 h at RT. Solution was dispelled and resin was washed with DMF (10x) and DCM (10x). Product was cleaved from resin by incubation with 500 µl of cleavage cocktail HFIP/DCM/TES 90/10/2 v/v/v for 10 min (3x). Resin was washed with 500 µl DCM (2x). TFA (250 µL) was added to the combined cleavage and washing solutions and the mixture was incubated at RT for 10 min. Volatiles were removed under reduced pressure. Residue was subjected to HPLC purification which gave thiols **3a/3b** as a mixture of the two regioisomers. HPLC eluate was used in the next step without further processing.

MS: m/z 678.1 = [M+H]⁺ (MW calculated = 678.0 g/mol).

### Treprostinil linker thiols 3c/3d

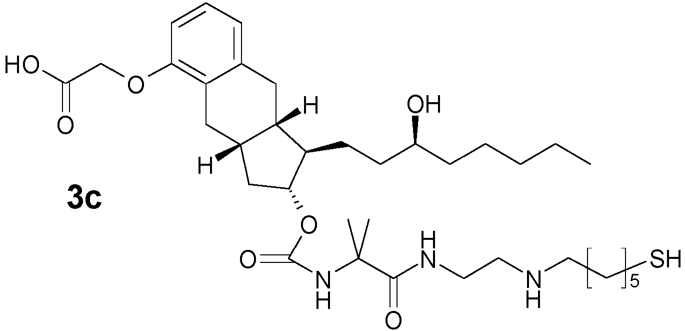

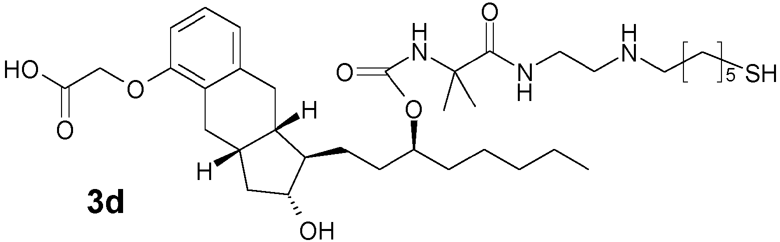

Treprostinil linker thiols **3c/3d** were synthesized as described for **3a/3b** except that linker building block **2b** was used instead of **2a.** Thiols **3c/3d** were obtained as a mixture of isomers. HPLC eluate was used in the next step without further processing.

MS: m/z 678.1 = [M+H]⁺ (MW calculated = 678.0 g/mol).

### Treprostinil linker thiols 3e and 3f

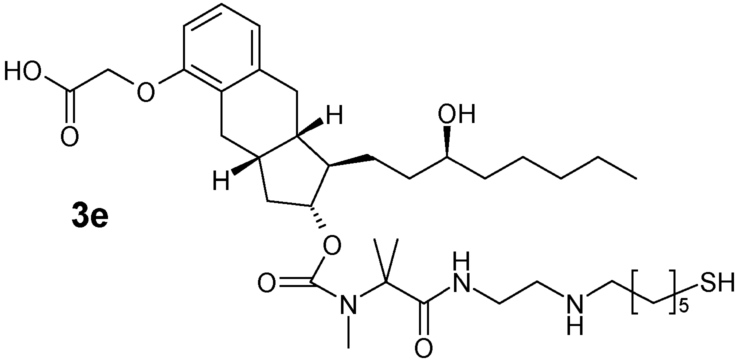

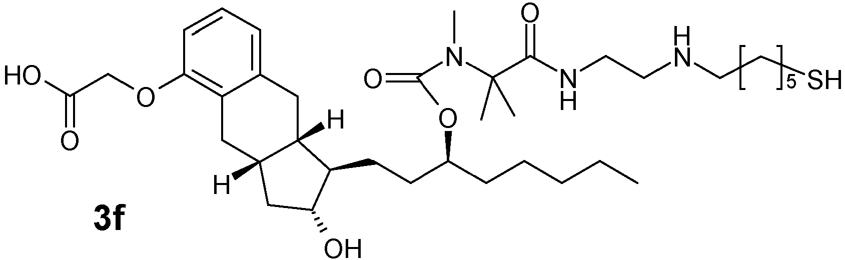

Treprostinil linker thiols **3e** and **3f** were synthesized as described for **3a/3b** except that linker building block **2c** was used instead of **2a.** Two isomers assigned to structures **3e** and **3f** were separated by HPLC. HPLC eluates were used in the next step without further processing.

**3e** MS: m/z 693.0 = [M+H]⁺ (MW calculated = 692.0 g/mol).

**3f** MS: m/z 693.0 = [M+H]⁺ (MW calculated = 692.0 g/mol).

### Example 4:

### Synthesis of linker building blocks 4a and 4b

Linker building blocks **4a** and **4b** were synthesized according to the following scheme:

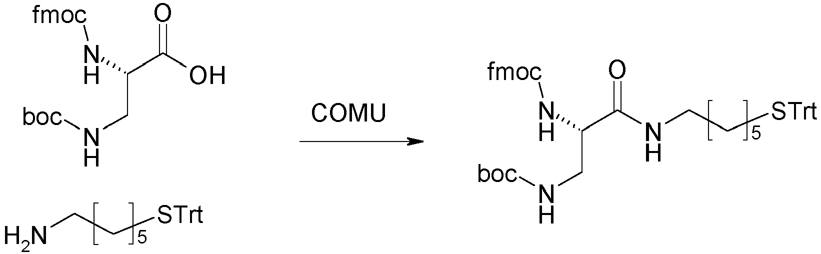

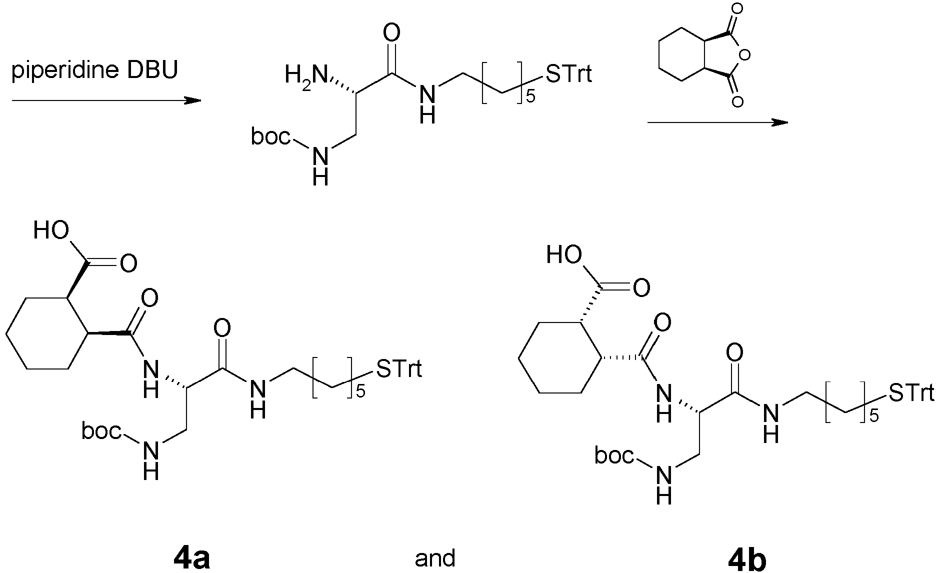

L-Fmoc-Dpr(Boc)-OH (100 mg, 0.234 mmol) was dissolved in 0.5 mL DMF (anhydrous, mol. sieve). 6-(*S*-Tritylsulfanyl)-hexaneamine (71 mg, 0.189 mmol), COMU (97 mg, 0.227 mmol) and DIPEA (66 µl, 0.378 mmol) were added and mixture was stirred for 1 h at RT. Piperidine (50 µl, 0.505 mmol) and DBU (40 µl, 0.336 mmol) were added and stirring was continued for 10 h. cis-Cyclohexanedicarboxylic anhydride (600 mg, 3.89 mmol) was added and stirring was continued for 1 h. Solution was quenched with water/acetonitrile and acidified with acetic acid. Building blocks were purified by RP-HPLC. Structures assignment of the earlier eluting diastereomer **4a** and the later eluting diastereomer **4b** was done arbitrarily and could also be reverse.

Yield: **4a** 30 mg (0.042 mmol), **4b** 42 mg (0.059 mmol)

MS: m/z 716.2 = [M+H]⁺ (MW calculated = 716.0 g/mol).

### Example 5:

### Synthesis of treprostinil linker thiols 5a/5b

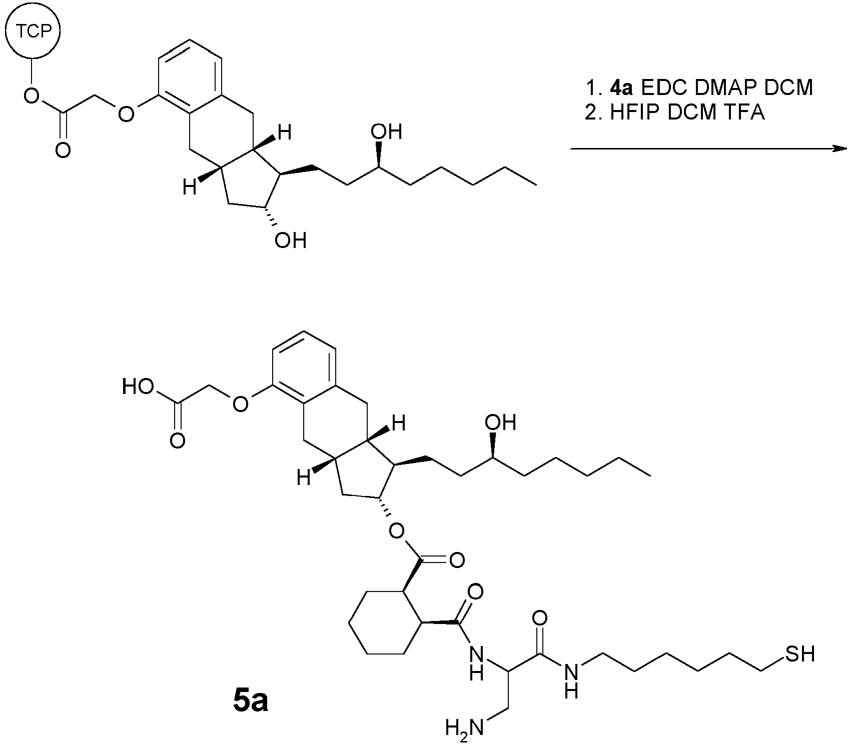

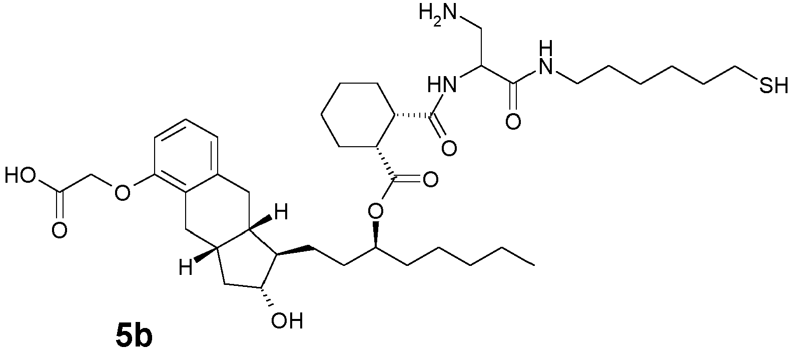

Linker building block **4a** (11 mg, 12 µmol), EDC HCl (7.4 mg, 38.5 µmol) and DMAP (4.7 mg, 38.5 µmol) were dissolved in 300 µl DCM (anhydrous, mol. sieve). Solution was drawn to 15 mg treprostinil loaded TCP resin (10.8 µmol, 0.72 mmol/g see Example 3) in a single use 2 mL syringe reactor equipped with a frit. Reactor was agitated for 15 h at RT. Solution was dispelled and resin was washed with DCM (10x). Product was cleaved by incubating resin with 500 µl HFIP/DCM 30/70 v/v for 10 min (3x). Resin was washed with 500 µl DCM (2x). To the combined cleavage and washing solutions were added 250 µl TFA and the mixture was incubated at RT for 10 min. Volatiles were removed under reduced pressure. Residue was subjected to RP-HPLC purification which gave thiols 5a/5b as a mixture of the two regioisomers. HPLC eluate was used in the next step without further processing.

Yield: 5a/5b 1.5 mg (2 µmol) as determined by thiol quantification by Ellman Test.

MS: m/z 746.2 = [M+H]⁺ (MW calculated = 746.0 g/mol).

### Example 6:

### Synthesis of PEG-linker-drug conjugates 6a/b, 6c/6d, 6e, 6f and 6g/6h

PEG-linker-drug conjugates were prepared according to the following scheme:

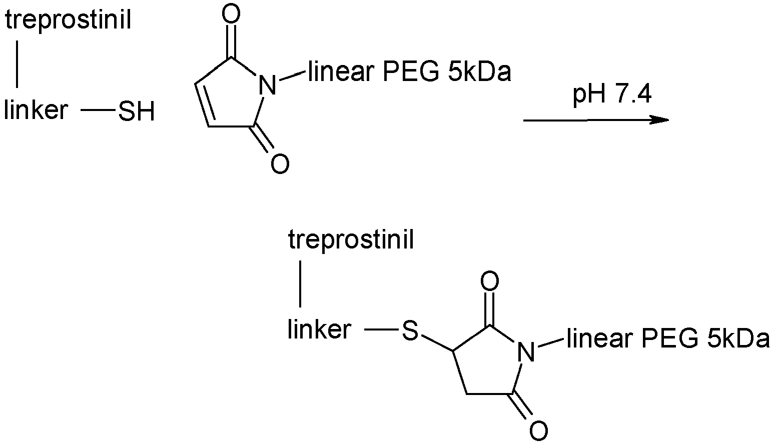

To HPLC eluates of treprostinil linker thiols **3a/3b, 3c/3d, 3e, 3f** and **5a/5b** was given an excess of linear 5 kDa PEG maleimide . Mixtures were neutralized by addition of pH 7.4 buffer (0.5 M phosphate) and incubated at RT. After complete consumption of thiol (approx. 1 h), mixtures were acidified with acetic acid and separated from excess PEG-maleimide by RP-HPLC. HPLC eluates were lyophilized to yield PEG-linker-drug conjugates **6a/b, 6c/6d, 6e, 6f** and **6g/6h** respectively.

### Example 7:

### Determination of drug release half life time from PEG conjugates 6a/b, 6c/6d, 6e, 6f and 6g/6h:

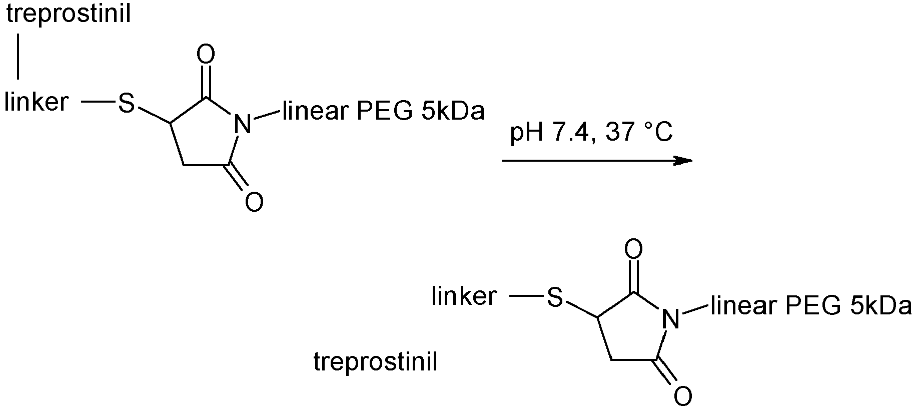

PEG-linker-drug conjugates **6a/b, 6c/6d, 6e, 6f** and **6g/6h** were dissolved in pH 7.4 buffer (60 mM sodium phosphate, 3 mM EDTA, 0.05 % Tween-20, 1 mL) and incubated at 37 °C. At various time points, aliquots were analyzed by UPLC to determine the amount of released treprostinil, which was plotted against time. Drug release was found to follow first order kinetics. Curve fitting software was used to determine half life time of drug release from the respective conjugates (Table 1)

**Table 1:**

| **entry** | **PEG-linker-drug conjugate** | **drug release half life time** |
|---|---|---|
| 1 | **6a/b** | 31 d |
| 2 | **6c/6d** | 17 d |
| 3 | **6e** | 24 d |
| 4 | **6f** | 37 d |
| 5 | **6g/6h** | 35 min |

### Abbreviations

- AcOH: Acetic acid
- AIB: 2-Aminoisobutyric acid
- Boc: tert-Butoxycarbonyl-
- BnBr: Benzylbromide
- BSA: *N*,*O*-Bis-(trimethylsilyl)-acetamid
- COMU: (1-Cyano-2-ethoxy-2-oxoethylidenaminooxy)dimethylamino-morpholino-carbenium hexafluorophosphate
- DIPEA: Diisopropylethylamine
- DCM: Dichloromethane
- DMAP: 4-(Dimethylamino)pyridine
- DMF: *N,N*-Dimethylformamide
- Dmob: 2,4-Dimethoxybenzyl
- DMSO: Dimethyl sulfoxide
- Dpr: 2,3-Diaminopropionic acid
- EDC: *N*-(3-Dimethylaminopropyl)-*N*'-ethylcarbodiimide
- EDTA: Ethylenediamine tetraacetic acid disodium salt dihydrate
- EtOAc: Ethyl acetate
- eq: Equivalent
- h: Hour
- HFIP: 1,1,1,3,3,3-Hexafluoroisopropanol
- HPLC: High performance liquid chromatography
- LC/MS: Mass spectrometry-coupled liquid chromatography
- m/z: Mass/charge
- Mal: Maleimido
- MeOH: Methanol
- min: Minute
- Mmt: 4-Methoxytriphenylmethyl
- mol.: Molecular
- NaOH: Sodium hydroxide
- NHS: *N*-Hydroxysuccinimide
- PEG: Polyethylene glycol
- Pfp: Pentafluorophenyl
- PyBOP: Benzotriazol-1-yl-oxytripyrrolidinophosphonium hexafluorophosphate
- RT: Room temperature
- RP: Reversed phase
- sat.: Saturated
- soln.: Solution
- T: Temperature
- TCP: 2-Chlorotrityl chloride resin
- TES: Triethylsilane
- Trt: Trityl
- THF: Tetrahydrofuran
- TFA: Trifluoroacetic acid
- UPLC: Ultra performance liquid chromatography
- UV: Ultra violet

## Claims

1. A prodrug or a pharmaceutically acceptable salt thereof comprising a biologically active moiety-linker conjugate D-L, wherein
D- is a hydroxyl-comprising biologically active moiety; and
-L is a promoiety comprising
i) a moiety -L¹- represented by formula (I), 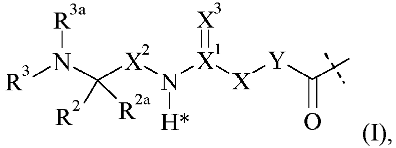
wherein the dashed line indicates attachment to a hydroxyl group of -D by forming an ester or carbamate bond;
-Y- is -C(R¹)(R^{1a})- or -N(R¹)-;
-X- is -C(R⁴)(R^{4a})-; 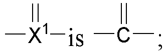
-X²- is -C(R⁷)(R^{7a})- or -C(R⁷)(R^{7a})-C(R⁸)(R^{8a})-;
=X³ is =O;
-R¹, -R^{1a}, -R², -R^{2a}, -R³, -R^{3a}, -R⁴, -R^{4a}, -R⁷, -R^{7a}, -R⁸, -R^{8a} are independently selected from the group consisting of -H and C₁₋₆ alkyl;
optionally, one or more of the pairs -R¹/-R^{1a}, -R²/-R^{2a}, -R²/-R³ , ⁻R⁴/-R^{4a}, -R⁷/-R^{7a}, -R⁷/-R⁸, -R⁸/-R^{8a} are joined together with the atom to which they are attached to form a ring T;
T is selected from the group consisting of phenyl; naphthyl; indenyl; indanyl; tetralinyl; C₃₋₁₀ cycloalkyl; 4- to 7-membered heterocyclyl; or 9- to 11-membered heterobicyc1yl, wherein T is optionally substituted with one or more -R⁹, which are the same or different;
-R⁹ is halogen, -CN, oxo (=O), -C(O)OH, -OH, -S(O)₂NH₂, -S(O)NH₂, -S(O)₂OH, -S(O)OH, -SH, -NH₂, -NO₂, C₁₋₆ alkyl or C₁₋₁₀ heteroalkyl;
optionally, -R³/-R^{3a} are joined together with the nitrogen atom to which they are attached to form a 4- to 7-membered heterocycle;
and
ii) a moiety -L²-, which is a chemical bond or a spacer, and -L²- is bound to a polymeric carrier group -Z,
wherein -L¹- is substituted with one -L²- moiety, provided that the hydrogen marked with the asterisk in formula (I) is not replaced by -L²-;
optionally, -L¹- is further substituted.

2. The prodrug or a pharmaceutically acceptable salt thereof of claim 1, wherein -Y- is -N(R¹)-.

3. The prodrug or a pharmaceutically acceptable salt thereof of claim 1 or 2, wherein -X²- is -C(R⁷)(R^{7a})-.

4. The prodrug or a pharmaceutically acceptable salt thereof of any one of claims 1 to 3, wherein -R¹, -R⁴ and -R^{4a} are independently selected from the group consisting of -H and methyl.

5. The prodrug or a pharmaceutically acceptable salt thereof of any one of claims 1 to 4, wherein -R¹ is methyl.

6. The prodrug or a pharmaceutically acceptable salt thereof of any one of claims 1 to 5, wherein -R⁴ and -R^{4a} are both methyl.

7. The prodrug or a pharmaceutically acceptable salt thereof of any one of claims 1 to 6, wherein -L¹- has the structure of formula (II): 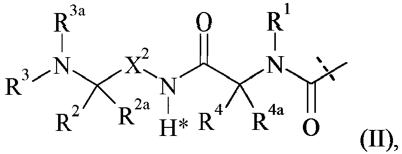
wherein the dashed line indicates attachment to a hydroxyl group of -D by forming a carbamate bond, and
-R¹, -R², -R^{2a}, -R³, -R^{3a}, -R⁴, -R^{4a} and -X²- have the meaning as indicated in any of claims 1 to 6.

8. The prodrug or a pharmaceutically acceptable salt thereof of any one of claims 1 to 7, wherein -L²- is C₁₋₅₀ heteroalkyl, which is optionally interrupted with one or more -T- and which is substituted with -Z, wherein -T- is selected from the group consisting of phenyl, naphthyl, indenyl, tetralinyl, C₃₋₁₀ cycloalkyl, 4- to 7-membered heterocyclyl and 9- to 11-membered heterobicyclyl; wherein -T- is optionally substituted with one or more -R⁹, which are the same or different; and -R⁹ is selected from the group consisting of halogen, -CN, oxo (=O), -C(O)OH; -OH, -S(O)₂NH₂, -S(O)NH₂, -S(O)₂OH, -S(O)OH, -SH, -NH₂, -NO₂, C₁₋₆ alkyl and C₁₋₁₀ heteroalkyl.

9. The prodrug or a pharmaceutically acceptable salt thereof of any one of claims 1 to 7, wherein -L²- is C₁₋₂₀ alkyl or C₁₋₂₀ heteroalkyl.

10. The prodrug or a pharmaceutically acceptable salt thereof of any one of claims 1 to 9, wherein -L²- has a molecular weight ranging from 14 g/mol to 750 g/mol.

11. The prodrug or a pharmaceutically acceptable salt thereof of any one of claims 1 to 10, wherein -Z is a polymer of at least 500 Da or a C₈₋₁₈ alkyl group.

12. The prodrug or a pharmaceutically acceptable salt thereof of any one of claims 1 to 11, wherein -Z is a polymer having a molecular weight of 0.5 to 160 kDa.

13. A pharmaceutical composition comprising the prodrug or a pharmaceutically acceptable salt thereof of any one of claims 1 to 12.

14. The prodrug or a pharmaceutically acceptable salt thereof of any one of claims 1 to 12 or the pharmaceutical composition of claim 13 for use as a medicament.

15. The prodrug or a pharmaceutically acceptable salt thereof of any one of claims 1 to 12 or the pharmaceutical composition of claim 13 for use in the treatment, control, delay or prevention of one or more conditions in a mammalian patient.

## Patentansprüche

1. Ein Prodrug oder ein pharmazeutisch verträgliches Salz davon, umfassend ein biologisch aktives Rest-Linker-Konjugat D-L, wobei
D- ein Hydroxyl-enthaltender biologisch aktiver Rest ist; und
-L eine Promoiety ist umfassend
i) einen Rest -L¹- der Formel (I), 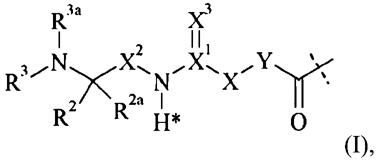
wobei die gestrichelte Linie die Anknüpfung an eine Hydroxylgruppe von -D durch die Bildung einer Ester- oder Carbamatbindung anzeigt;
-Y- -C(R¹)(R^{1a})- oder -N(R¹)- ist;
-X- -C(R⁴)(R^{4a})- ist; 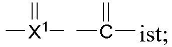
-X²- -C(R⁷)(R^{7a})- oder -C(R⁷)(R^{7a})-C(R⁸)(R^{8a})- ist;
=X³ =O ist;
-R¹, -R^{1a}, -R², -R^{2a}, -R³, -R^{3a}, -R⁴, -R^{4a}, -R⁷, -R^{7a}, -R⁸, -R^{8a} unabhängig ausgewählt sind aus der Gruppe bestehend aus -H und C₁₋₆-Alkyl;
optional eines oder mehrere der Paare -R¹/-R^{1a}, -R²/-R^{2a}, -R²/-R³, -R⁴/-R^{4a}, -R⁷/-R^{7a}, -R⁷/-R⁸, -R⁸/-R^{8a} zusammen mit den Atomen, an die sie gebunden sind, verbunden sind und einen Ring T formen;
T ausgewählt ist aus der Gruppe bestehend aus Phenyl; Naphthyl; Indenyl; Indanyl; Tetralinyl; C₃₋₁₀-Cycloalkyl; 4- bis 7-gliedrigem Heterocyclyl; oder 9-bis 11-gliedrigem Heterobicyclyl, wobei T optional mit einem oder mehreren -R⁹ substituiert ist, die gleich oder unterschiedlich sind;
-R⁹ Halogen, -CN, oxo (=O), -C(O)OH, -OH, -S(O)₂NH₂, -S(O)NH₂, -S(O)₂OH, -S(O)OH, -SH, -NH₂, -NO₂, C₁₋₆ Alkyl oder C₁₋₁₀ Heteroalkyl ist;
optional -R³/-R^{3a} zusammen mit dem Stickstoffatom, an das sie gebunden sind, verbunden sind und einen 4- bis 7-gliedrigen Heterocyclus formed;
und
ii) ein Rest -L²-, welcher eine chemische Bindung oder ein Abstandshalter ist, und wobei -L²- an eine polymere Trägergruppe -Z gebunden ist,
wobei -L¹- mit einem -L²- Rest substituiert ist, vorausgesetzt, dass der mit einem Asterisk markierte Wasserstoff in Formel (I) nicht durch -L²- ersetzt wird;
optional ist -L¹- weiter substituiert.

2. Das Prodrug oder ein pharmazeutisch verträgliches Salz davon nach Anspruch 1, wobei -Y- -N(R¹)- ist.

3. Das Prodrug oder ein pharmazeutisch verträgliches Salz davon nach Anspruch 1 oder 2, wobei -X²- -C(R⁷)(R^{7a})- ist.

4. Das Prodrug oder ein pharmazeutisch verträgliches Salz davon nach einem der Ansprüche 1 bis 3, wobei -R¹, -R⁴ und -R^{4a} unabhängig voneinander ausgewählt sind aus der Gruppe bestehend aus -H und Methyl.

5. Das Prodrug oder ein pharmazeutisch verträgliches Salz davon nach einem der Ansprüche 1 bis 4, wobei -R¹ Methyl ist.

6. Das Prodrug oder ein pharmazeutisch verträgliches Salz davon nach einem der Ansprüche 1 bis 5, wobei -R⁴ und -R^{4a} beide Methyl sind.

7. Das Prodrug oder ein pharmazeutisch verträgliches Salz davon nach einem der Ansprüche 1 bis 6, wobei -L¹- der Formel (II) entspricht: 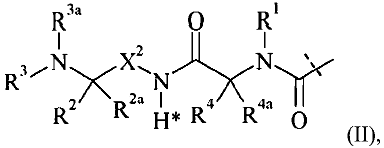
wobei die gestrichelte Linie die Anknüpfung an eine Hydroxylgruppe von -D durch die Bildung einer Carbamatbindung anzeigt, und
-R¹, -R², -R^{2a}, -R³, -R^{3a}, -R⁴, -R^{4a} und -X²- die Bedeutung wie in einem der Ansprüche 1 bis 6 angegeben haben.

8. Das Prodrug oder ein pharmazeutisch verträgliches Salz davon nach einem der Ansprüche 1 bis 7, wobei -L²- C₁₋₅₀-Heteroalkyl ist, welches optional mit einem oder mehreren -T- unterbrochen ist und welches mit -Z substituiert ist, wobei -T- ausgewählt ist aus der Gruppe bestehend aus Phenyl, Naphthyl, Indenyl, Tetralinyl, C₃₋₁₀-Cycloalkyl, 4- bis 7-gliedrigem Heterocyclyl und 9- bis 11-gliedrigem Heterobicyclyl; wobei -T- optional mit einem oder mehreren -R⁹ substituiert ist, welche gleich oder unterschiedlich sind; und -R⁹ ausgewählt ist aus der Gruppe bestehend aus Halogen, -CN, oxo (=O), -C(O)OH, -OH, -S(O)₂NH₂, -S(O)NH₂, -S(O)₂OH, -S(O)OH, -SH, -NH₂, -NO₂, C₁₋₆ Alkyl und C₁₋₁₀ Heteroalkyl.

9. Das Prodrug oder ein pharmazeutisch verträgliches Salz davon nach einem der Ansprüche 1 bis 7, wobei -L²- C₁₋₂₀ Alkyl oder C₁₋₂₀ Heteroalkyl ist.

10. Das Prodrug oder ein pharmazeutisch verträgliches Salz davon nach einem der Ansprüche 1 bis 9, wobei -L²- ein Molekulargewicht im Bereich von 14 g/mol bis 750 g/mol hat.

11. Das Prodrug oder ein pharmazeutisch verträgliches Salz davon nach einem der Ansprüche 1 bis 10, wobei -Z ein Polymer mit mindestens 500 Da oder eine C₈₋₁₈ Alkyl Gruppe ist,

12. Das Prodrug oder ein pharmazeutisch verträgliches Salz davon nach einem der Ansprüche 1 bis 11, wobei -Z ein Polymer mit einem Molekulargewicht von 0.5 bis 160 kDa ist.

13. Eine pharmazeutische Zusammensetzung umfassend das Prodrug oder ein pharmazeutisch verträgliches Salz davon nach einem der Ansprüche 1 bis 12.

14. Das Prodrug oder ein pharmazeutisch verträgliches Salz davon nach einem der Ansprüche 1 bis 12 oder die pharmazeutische Zusammensetzung nach Anspruch 13 zur Verwendung als Medikament.

15. Das Prodrug oder ein pharmazeutisch verträgliches Salz davon nach einem der Ansprüche 1 bis 12 oder die pharmazeutische Zusammensetzung nach Anspruch 13 zur Verwendung in der Behandlung, Kontrolle, Verzögerung oder Prävention von einer oder mehrerer Erkrankungen in einem Säugetierpatienten.

## Revendications

1. Promédicament ou sel pharmaceutiquement acceptable de celui-ci comprenant un conjugué D-L fraction biologiquement active-lieur, dans lequel
D- est une fraction biologiquement active comprenant un hydroxyle ; et
-L est une fraction précurseur comprenant
i) une fraction -L¹- représentée par la formule (I), 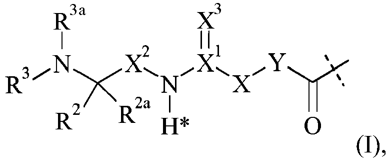
la ligne pointillée indiquant l'attache à un groupe hydroxyle de -D par formation d'une liaison ester ou carbamate ;
-Y- étant -C(R¹)(R^{1a})- ou -N(R¹)- ;
-X- étant -C(R⁴)(R^{4a})- ; 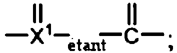
-X²- étant -C(R⁷)(R^{7a})- ou -C(R⁷)(R^{7a})-C(R⁸)(R^{8a})- ;
=X³ étant =O ;
-R¹, -R^{1a}, -R², -R^{2a}, -R³, -R^{3a}, -R⁴, -R^{4a}, -R⁷, -R^{7a}, -R⁸, -R^{8a} étant indépendamment choisis dans le groupe constitué par -H et alkyle en C₁₋₆ ;
éventuellement, une ou plusieurs des paires -R¹/-R^{1a}, -R²/-R^{2a}, -R²/-R³, - R⁴/-R^{4a}, -R⁷/-R^{7a}, -R⁷/-R⁸, -R⁸/-R^{8a} étant unies conjointement avec l'atome auquel elles sont attachées pour former un cycle T ;
T étant choisi dans le groupe constitué par phényle ; naphtyle ; indényle ; indanyle ; tétralinyle ; cycloalkyle en C₃₋₁₀ ; hétérocyclyle à 4 à 7 chaînons ; ou hétérobicyclyle à 9 à 11 chaînons, T étant éventuellement substitué par un ou plusieurs -R⁹, qui sont identiques ou différents ;
-R⁹ étant halogéno, -CN, oxo (=O), -C(O)OH, -OH, -S(O)₂NH₂, -S(O)NH₂, -S(O)₂OH, -S(O)OH, -SH, -NH₂, -NO₂, alkyle en C₁₋₆ ou hétéroalkyle en C₁₋₁₀ ;
éventuellement, -R³/-R^{3a} étant unis conjointement avec l'atome d'azote auquel ils sont attachés pour former un hétérocycle à 4 à 7 chaînons ;
et
ii) une fraction -L²-, qui est une liaison chimique ou un groupe d'espacement et -L²- étant lié à un groupe porteur polymère -Z,
-L¹- étant substitué par une fraction -L²- à condition que l'hydrogène marqué par l'astérisque dans la formule (I) ne soit pas remplacé par -L²- ;
éventuellement, -L¹- étant encore substitué.

2. Promédicament ou sel pharmaceutiquement acceptable de celui-ci selon la revendication 1, dans lequel -Y- est -N(R¹)-.

3. Promédicament ou sel pharmaceutiquement acceptable de celui-ci selon la revendication 1 ou 2, dans lequel -X²- est -C(R⁷)(R^{7a})-.

4. Promédicament ou sel pharmaceutiquement acceptable de celui-ci selon l'une quelconque des revendications 1 à 3, dans lequel R¹, -R⁴ et -R^{4a} sont indépendamment choisis dans le groupe constitué par -H et méthyle.

5. Promédicament ou sel pharmaceutiquement acceptable de celui-ci selon l'une quelconque des revendications 1 à 4, dans lequel -R¹ est méthyle.

6. Promédicament ou sel pharmaceutiquement acceptable de celui-ci selon l'une quelconque des revendications 1 à 5, dans lequel -R⁴ et -R^{4a} sont tous deux méthyle.

7. Promédicament ou sel pharmaceutiquement acceptable de celui-ci selon l'une quelconque des revendications 1 à 6, dans lequel -L¹- a la structure de formule (II) : 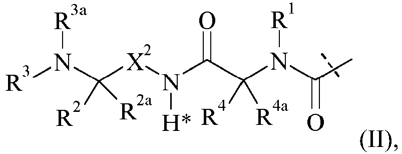
la ligne pointillée indiquant l'attache à un groupe hydroxyle de -D par formation d'une liaison carbamate et
-R¹, -R², -R^{2a}, -R³, -R^{3a}, -R⁴, -R^{4a} et -X²- ayant la signification telle qu'indiquée dans l'une quelconque des revendications 1 à 6.

8. Promédicament ou sel pharmaceutiquement acceptable de celui-ci selon l'une quelconque des revendications 1 à 7, dans lequel -L²- est hétéroalkyle en C₁₋₅₀, qui est éventuellement interrompu par un ou plusieurs -T- et qui est substitué par -Z, -T- étant choisi dans le groupe constitué par phényle, naphtyle, indényle, tétralinyle, cycloalkyle en C₃₋₁₀, hétérocyclyle à 4 à 7 chaînons et hétérobicyclyle à 9 à 11 chaînons ; -T- étant éventuellement substitué par un ou plusieurs -R⁹, qui sont identiques ou différents ; et -R⁹ étant choisi dans le groupe constitué par halogéno, -CN, oxo (=O), -C(O)OH ; -OH, -S(O)₂NH₂, -S(O)NH₂, -S(O)₂OH, -S(O)OH, -SH, -NH₂, -NO₂, alkyle en C₁₋₆ et hétéroalkyle en C₁₋₁₀.

9. Promédicament ou sel pharmaceutiquement acceptable de celui-ci selon l'une quelconque des revendications 1 à 7, dans lequel -L²- est alkyle en C₁₋₂₀ ou hétéroalkyle en C₁₋₂₀.

10. Promédicament ou sel pharmaceutiquement acceptable de celui-ci selon l'une quelconque des revendications 1 à 9, dans lequel -L²- a une masse moléculaire allant de 14 g/mol à 750 g/mol.

11. Promédicament ou sel pharmaceutiquement acceptable de celui-ci selon l'une quelconque des revendications 1 à 10, dans lequel -Z est un polymère d'au moins 500 Da ou un groupe alkyle en C₈₋₁₈.

12. Promédicament ou sel pharmaceutiquement acceptable de celui-ci selon l'une quelconque des revendications 1 à 11, dans lequel -Z- est un polymère ayant une masse moléculaire de 0,5 à 160 kDa.

13. Composition pharmaceutique comprenant le promédicament ou un sel pharmaceutiquement acceptable de celui-ci selon l'une quelconque des revendications 1 à 12.

14. Promédicament ou sel pharmaceutiquement acceptable de celui-ci selon l'une quelconque des revendications 1 à 12 ou composition pharmaceutique selon la revendication 13 destinés à être utilisés en tant que médicament.

15. Promédicament ou sel pharmaceutiquement acceptable de celui-ci selon l'une quelconque des revendications 1 à 12 ou composition pharmaceutique selon la revendication 13 destinés à être utilisés dans le traitement d'une ou plusieurs affections, le contrôle de celles-ci, le retardement de celles-ci ou la prévention de celles-ci chez un patient mammifère.
